(19) Europäisches Patentamt
European Patent Office
Office européen des brevets

(11) EP 4 180 529 A1

(12) EUROPEAN PATENT APPLICATION

(43) Date of publication:
**17.05.2023 Bulletin 2023/20**

(21) Application number: **21207910.7**

(22) Date of filing: **12.11.2021**

(51) International Patent Classification (IPC):
***C12P 7/10*** (2006.01) ***C12P 7/08*** (2006.01)
***C12N 1/22*** (2006.01)

(52) Cooperative Patent Classification (CPC):
**C12P 7/10; C12N 1/22; C12P 7/08;** C12P 2203/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Sekab E-Technology AB**
**891 26 Örnsköldsvik (SE)**

(72) Inventors:
- **SIBBESEN, Ole**
  **2880 BAGSVAERD (DK)**
- **CAVKA, Adnan**
  **891 96 ARNÄSVALL (SE)**

(74) Representative: **Kransell & Wennborg KB**
**P.O. Box 27834**
**115 93 Stockholm (SE)**

# (54) METHOD FOR A MICROBIAL FERMENTATION PROCESS

(57) The present invention relates to method for a microbial fermentation process involving fermentation of a fermentation media derived from biomass to a target fermentation product. The method comprising: providing a reference curve defined by a predetermined weight ratio of galactose to xylose as a function of acetic acid concentration present in the fermentation media; providing a first biomass-based stream and a second biomass-based stream different to the first biomass-based stream, comprising galactose and xylose; mixing the first and second biomass-based streams; providing a fermentation media by treating the mixed first and second biomass-based streams, or treating the first and second biomass-based streams separately prior to the mixing, wherein the mixing of the first and second biomass-based streams results in a weight ratio of galactose to xylose in the fermentation media closer to the reference curve compared to at least one of the first and second biomass-based streams; and fermenting the fermentation media by means of fermentation microorganisms, such that the xylose and galactose are simultaneously fermented into the same target fermentation product.

S2
S6
S4
S7
S5
S10
S20
S30
S40

Fig. 3

EP 4 180 529 A1

## Description

### TECHNICAL FIELD

The present disclosure generally relates to a method for a microbial fermentation process involving fermentation of a fermentation media derived from biomass to a target fermentation product. In particular, the invention relates to fermenting a fermentation media comprising at least xylose and galactose.

### BACKGROUND

Biomass residues and waste from forestry are attractive as feedstocks for the production of green chemicals and fuels, since they are abundant, relatively inexpensive, and not used for food. Those residues consist mainly of lignin and two classes of polysaccharides, cellulose and hemicellulose. Cellulose is composed of polysaccharide chains of several hundred to over ten thousand linked glucose units, whereas hemicellulose is a polysaccharide composed of xylose, other pentose sugars and various hexose sugars, e.g. glucose and mannose. In lignocellulose, cellulose and hemicellulose are tightly associated to lignin, a polyphenolic compound that ties the cellulose and hemicellulose polymers together, thus providing the lignocellulose with rigidity and mechanical strength. Other abundant sources of biomass, such as e.g. agricultural waste and algae, are also attractive as feedstocks for the production of green chemicals and fuels.

For example, in the production of ethanol from lignocellulosic materials, various pretreatment and hydrolysis steps are typically used to separate cellulose, hemicellulose and lignin and subsequently to degrade the cellulose and hemicellulose polysaccharides to fermentable saccharides in a fermentation media, often referred to as "hydrolysate". The fermentable saccharides are then converted to ethanol by means of fermenting microorganisms, such as yeast or bacteria, and the ethanol is recovered by means of distillation. The yeast Saccharomyces cerevisiae (S. cerevisiae), for example, metabolizes hexose sugars and is a microorganism suitable for industrial processes for cellulosic bioethanol production. However, S. cerevisiae does not naturally ferment xylose that comprises a major part of many types of biomass derived material, why much attention has been put forth to genetic engineering of yeast to also possess the ability to ferment xylose to ethanol and therefore to more fully utilize the available sugars from lignocellulose. This has been accomplished with two different genetic engineering strategies: 1) to insert into the yeast a gene that enables the expression of a xylose isomerase enzyme that enables the yeast to intracellular conversion of xylose into xylulose, or 2) to insert into the yeast two genes that enables the expression of a xylose reductase enzyme and a xylitol dehydrogenase enzyme, the combined action of the two enzymes enables the yeast to intracellular conversion of xylose into xylulose.

However, with engineered yeast being able to ferment xylose, the fermenting rate of xylose is significantly slower than that of e.g. glucose. In addition, other sugars which can be metabolized by S. cerevisiae, are not as efficiently metabolized as glucose. For example, galactose, which is slightly different from glucose in its conformation, requires additional enzymes and cofactors in its metabolism, implying that additional energy is required before the energy from the metabolism can be harvested. This again means that the net gained energy is lower than it is for glucose. Thus, the yeast has evolved a repression mechanism that saves energy by suppressing the galactose metabolism very tightly when there is glucose, fructose or mannose present.

There is thus a need in the industry to more efficiently ferment biomass-based fermentation media containing various different sugar, and/or to increase the yield of the target fermentation product for such fermentation.

### SUMMARY

In view of the above, it is an object of the present invention to provide improvements with respect to methods and arrangements for fermentation of biomass-based derived material, particularly to achieve a more favourable process for the fermentation of xylose and galactose.

According to a first aspect of the invention, a method for a microbial fermentation process involving fermentation of a fermentation media derived from biomass to a target fermentation product is provided. The method comprises:

- providing a reference curve defined by a predetermined weight ratio of galactose to xylose as a function of acetic acid concentration present in the fermentation media,
- providing a first biomass-based stream and a second biomass-based stream different to the first biomass-based stream, wherein at least one of the first and second biomass-based streams has a weight ratio of galactose to xylose as a function of acetic acid concentration below the reference curve;
- mixing the first and second biomass-based streams;
- providing a fermentation media by treating the mixed first and second biomass-based streams, or treating the first and second biomass-based streams separately prior to the mixing, wherein the mixing of the first and second biomass-based streams results in a weight ratio of galactose to xylose in the fermentation media closer to the reference curve compared to at least one of the first and second biomass-based streams; and

- fermenting the fermentation media by means of fermentation microorganisms, such that the xylose and galactose are simultaneously fermented into the same target fermentation product.

Hereby, a more favourable process for the fermentation of xylose and galactose is provided. The method results in an improved usage of the first and second biomass-based streams. Stated differently, the fermentation of galactose and xylose is improved owing to the mixing of the first and second biomass-based streams. Thus, the first and second biomass-based streams together comprises galactose and xylose. Stated differently, the provided fermentation media comprises galactose and xylose. Any ratio of galactose to xylose is typically based on weight (w/w), and may e.g. be derived by taking a ratio of the galactose concentration (g/L) to the xylose concentration (g/L). The concentration is in the unit g/L if nothing else is stated.

According to at least one example embodiment, the predetermined weight ratio of galactose to xylose is a target weight ratio, and the reference curve is thus defined by the target weight ratio of galactose to xylose as a function of acetic acid concentration (g/L) present in the fermentation media. According to at least one example embodiment, the target weight ratio it at least 0.1, such as at least 0.12 or at least 0.15, or at least 0.166. Thus, according to at least one example, the minimum value of the reference curve is 0.1, or 0.12, or 0.15 or 0.166. According to at least one example embodiment, the reference curve is referred to as an optimal reference curve, or a beneficial curve. According to at least one example embodiment, the mixing of the first and second biomass-based streams results in a weight ratio of galactose to xylose in the fermentation media closer to the area above the reference curve compared to at least one of the first and second biomass-based streams.

According to at least one example embodiment, the first biomass-based stream is derived from a first raw biomass material, and the second biomass-based stream is derived from a second raw biomass material being different to the first raw biomass material. For example, the first and second raw biomass materials are of different types, or are derived from different types of biomass, or are derived from different types of trees.

It should be understood that for embodiments in which the fermentation media is provided by treating the first and second biomass-based streams separately prior to the mixing, the treatment of the separated first and second biomass-based streams are followed by said step of mixing.

According to at least one example embodiment, the first biomass-based stream comprises galactose and xylose at a first stream galactose to xylose weight ratio, and/or the second biomass-based stream comprises galactose and xylose at a second stream galactose to xylose weight ratio, wherein the weight ratio of galactose to xylose in the fermentation media is closer to the reference curve (or the area above the reference curve) compared to the first stream galactose to xylose weight ratio and/or the second stream galactose to xylose weight ratio. Any stream galactose to xylose ratio is typically based on weight (w/w).

It should be understood that any weight ratio of galactose to xylose (e.g. in the first biomass-based stream and/or the second biomass-based stream) is typically defined for a given acetic acid concentration (e.g. for a specific point), or is a function of the acetic acid concentration (e.g. for a curve).

By providing a weight ratio of galactose to xylose in the fermentation media closer to the reference curve (or the area above the reference curve), the fermentation of galactose and xylose is improved at least compared to fermenting a respective fermentation media corresponding to the individual first and second biomass-based streams.

According to at least one example embodiment, the first stream galactose to xylose weight ratio and the second stream galactose to xylose weight ratio are arranged on opposite sides of the reference curve. That is, the method may comprise using a first raw biomass material and a second raw biomass material resulting in that the first stream galactose to xylose weight ratio and the second stream galactose to xylose weight ratio are arranged on opposite sides of the reference curve

According to at least one example embodiment, the reference curve is defined as $y = A + B * e^{(x/C)}$ wherein y is the threshold beneficial weight ratio of galactose to xylose (w/w) and x is the concentration of acetic acid (g/L) in the fermentation media, and wherein A, B and C are constants.

According to at least one example embodiment, A is 0.07 +/- 0.06, B is 0.03 +/- 0.02 and C is 2.35 +/- 0.25. According to at least one example embodiment A is 0.07, B is 0.03 and C is 2.35, typically at a pH of 5.5.

According to at least one example embodiment, the method further comprises:

- determining the galactose to xylose weight ratio in the fermentation media;
- in response of determining that the galactose to xylose weight ratio in the fermentation media is below the reference curve, adjusting the proportion of the first and second biomass-based streams in order to adjust the galactose to xylose weight ratio to be above the reference curve.

Hereby, a simple yet effective means for adjusting the galactose to xylose weight ratio relative to the reference curve, thereby adapting the galactose to xylose weight ratio to be above the reference curve, is provided. The determined galactose to xylose weight ratio in the fermentation media may be referring to the initial galactose to xylose weight ratio prior to fermentation. Alternatively, the method comprises in response of determining that the galactose to xylose weight ratio in the fermentation media is offset compared to the reference curve, adjusting the proportion of the first and second biomass-based streams in order to adjust the galactose to xylose weight ratio to be closer to the reference curve.

According to a second aspect of the present invention, a method for a substantial anaerobic microbial fermentation process involving fermentation of a fermentation media derived from biomass to a target fermentation product is provided. The method comprises:

- providing a fermentation media comprising at least xylose and galactose;
- fermenting the fermentation media by means of fermentation microorganisms, such that the xylose and galactose are simultaneously fermented into the same target fermentation product, wherein the fermenting is performed in the presence of acetic acid.

Hereby, the simultaneously fermentation of xylose and galactose is improved. The presence of acetic acid during the fermentation promotes the simultaneous fermentation of xylose and galactose such that the fermentation rate and/or the yield of the target fermentation product is improved. Thus, the method of the second aspect of the invention comprises a substantial anaerobic microbial fermentation process involving fermentation of a sugar containing fermentation media, derived and prepared from one or more sources of biomass. Effects and features of the second aspect of the invention, at least related to the simultaneous fermentation of xylose and galactose, are largely analogous to those described above in connection with the first aspect of the invention. Embodiments mentioned in relation to the first aspect of the invention are largely compatible with the second aspect of the invention, of which some are exemplified below. For example, any ratio of galactose to xylose is typically based on weight (w/w), and may e.g. be derived by taking a ratio of the galactose concentration (g/L) to the xylose concentration (g/L). The concentration is in the unit g/L if nothing else is stated.

According to at least one example embodiment, the method further comprises:

- providing a first biomass-based stream and a second biomass-based stream different to the first biomass-based stream, the first and second biomass-based streams together comprising xylose and galactose,
- mixing the first and second biomass-based streams;
- providing a fermentation media by treating the mixed first and second biomass-based streams, or treating the first and second biomass-based streams separately prior to the mixing, wherein the mixing of the first and second biomass-based streams results in a weight ratio of galactose to xylose in the fermentation media within a target range.

Hereby, the preferred weight ratio of galactose to xylose in the fermentation media can be reached by mixing at least two biomass-based streams. According to at least one example embodiment, the first biomass-based stream is derived from a first raw biomass material, and the second biomass-based stream is derived from a second raw biomass material being different to the first raw biomass material. Thus, at least two separate biomass-based streams are mixed to reach a target range of the weight ratio of galactose to xylose in the fermentation media. The fermentation is improved by the simultaneous fermentation of xylose and galactose, and in particular for a specific relative amounts or concentrations of galactose to xylose. The target range may according to one example embodiment comprise a range around the target ratios of the reference curve of the first aspect of the invention. According to at least one example embodiment, the lower limit of the target range 0.1, such as 0.12, or 0.15, or 0.166. This is e.g. applicable for an acetic acid concentration of at least 2 g/L at pH 5.5. The higher limit of the target range may e.g. be 3.0.

According to at least one example embodiment, the weight ratio of galactose to xylose in the fermentation media within the target range is referring to a condition at the start of, or prior to, the fermentation of the fermentation media. Thus, such weight ratio of galactose to xylose may be referred to as an initial weight ratio of galactose to xylose in the fermentation media, and the target range may be referred to as an initial target range.

According to at least one example embodiment, the weight ratio of galactose to xylose in the fermentation media within the target range is referring to a condition during the fermentation of the fermentation media, applicable e.g. during a continuous fermentation process.

According to at least one example embodiment, the target range of the weight ratio of galactose to xylose in the fermentation media is above 0.1, such as above 0.12, or above 0.15, or above 0.166, such as e.g. between 0.166 and 3.

According to at least one example embodiment, the first biomass-based stream comprises xylose and galactose at a first stream galactose to xylose weight ratio, and/or the second biomass-based stream comprises xylose and galactose at a second galactose to xylose stream weight ratio, wherein the weight ratio of galactose to xylose in the fermentation media is closer to a desired weight ratio compared to the first stream galactose to xylose weight ratio and/or the second galactose to xylose stream weight ratio. The first stream galactose to xylose weight ratio may simply be referred to as the first stream weight ratio, and the second stream galactose to xylose weight ratio may simply be referred to as the second stream weight ratio. Any stream ratio is typically based on weight (w/w).

According to at least one example embodiment, the method comprises:

- determining the galactose to xylose weight ratio in the fermentation media;
- in response of determining that the galactose to xylose weight ratio in the fermentation media is outside of the target range, adjusting the proportion of the first and second biomass-based streams in order to adjust the galactose to xylose weight ratio towards the target range.

Hereby, a simple yet effective means for reaching the target range of galactose to xylose weight ratio, or at least adapting the galactose to xylose weight ratio to be closer to the target range, is provided. The determined galactose to xylose weight ratio in the fermentation media may be referring to the initial galactose to xylose weight ratio prior to fermentation.

The simultaneous fermentation of xylose into the same target fermentation product may be achieved by mixing the first and second biomass-based streams with consideration to a reference curve as described with reference to the first aspect of the invention, or it may be achieved by mixing the first and second biomass-based streams with consideration to a target range of the weight ratio of galactose to xylose in the fermentation media.

The following embodiments are applicable to the first aspect and the second aspect of the invention. In particular, the embodiments related to the first biomass-based stream and the second biomass-based stream are applicable for both the first and second aspects of the invention. It should be understood that according to at least one example embodiment, the fermenting of the fermentation media in the first aspect of the invention is performed in the presence of acetic acid, and that the fermentation may be a substantial anaerobic microbial fermentation process as in the second aspect of the invention.

According to at least one example embodiment, mixing of the at least two biomass-based streams results in a weight ratio of galactose to xylose in the fermentation media which is improved compared to the galactose to xylose weight ratio of the individual first and/or second biomass-based streams (referred to as a first stream weight ratio and a second stream weight ratio, respectively). Thus, the galactose to xylose weight ratio in the fermentation media may be adapted by the varying the xylose and/or galactose content in the first and/or second biomass-based streams (e.g. by choosing different biomass material), and/or by varying the degree of mixing of the first and second biomass-based streams. In other words, by varying or choosing the first stream weight ratio and/or the second stream weight ratio, and/or the degree of mixing of the first and second biomass-based streams, the weight ratio of galactose to xylose in the fermentation media can be controlled. Hereby, the desired weight ratio of galactose to xylose in the fermentation media can be reached, the desired weight ratio being closer to the reference curve (or the area above the reference curve) compared to at least one of the first and second biomass-based streams, or be a weight ratio within the target range. It should be noted that the weight ratio of galactose to xylose in the fermentation media and the desired weight ratio may be referring to a condition at the start of, or prior to, the fermentation of the fermentation media, and/or the weight ratio of galactose to xylose in the fermentation media and the desired weight ratio may be referring to a condition during the fermentation of the fermentation media, applicable e.g. during a continuous fermentation process.

According to at least one example embodiment, the first biomass-based stream has a weight ratio (w/w) of galactose to xylose between 0.3 and 1.7, such as e.g. between 0.34 and 1.68 (or between 0.338 and 1.68). According to at least one example, the second biomass-based stream has a weight ratio (w/w) of galactose to xylose between 0.05 and 0.2, such as between 0.07 and 0.15 (or between 0.07 and 0.105). That is, according to at least one example embodiment, the first stream weight ratio is between 0.3 and 1.7, such as e.g. between 0.34 and 1.68 (or between 0.338 and 1.68). According to at least one example embodiment, the second stream weight ratio is between 0.05 and 0.2, such as between 0.07 and 0.15 (or between 0.07 and 0.105). According to at least one example embodiment, the desired weight ratio of galactose to xylose in the fermentation media is above 0.1, such as above 0.12, or above 0.15, or above 0.166.

For example, the first raw biomass material is chosen from: Fir (e.g. Douglas Fir), Spruce (e.g. Norway spruce) and Pine (e.g. Radiate pine or Lobolly pine). For example, the second raw biomass material is chosen from: Corn stover and What straw. For example, Fir (e.g. Douglas Fir) and Corn stover is mixed in a proportion of at least 1:4, or a proportion of at least 1:3, or a proportion of at least 1:2, or a proportion of at least 1:1, such as e.g. a proportion of between 1:1 and 1:4, or between 1:1 and 1:3, or between 1:1 and 1:2 (weight, total dry matter). According to another example, Spruce (e.g. Norway spruce) and Wheat straw is mixed in a proportion of at least 1:1, or a proportion of at least 2:1, or a proportion of at least 3:1, or a proportion of at least 4:1, such as e.g. a proportion of between 1:1 to 4:1, or between 2:1 to 4:1 (weight, total dry matter). According to another example, Radiate pine and Corn stover is mixed in a proportion of at least 1:4, or a proportion of at least 1:3, or a proportion of at least 1:2, or a proportion of at least 1:1, such as e.g. a proportion of between 1:1 to 1:4, or between 1:1 to 1:3, or between 1:1 to 1:2 (weight, total dry matter). According to another example, Lobolly pine and Wheat straw is mixed in a proportion of at least 1:1, or a proportion of at least 2:1, or a proportion of at least 3:1, or a proportion of at least 4:1, such as e.g. a proportion of between 1:1 to 4:1 (weight, total dry matter). All mixing proportion are based on weight, total dry matter (w/w), and the proportion ratio between the ingoing raw biomass material may be referred to as a weight ratio of the ingoing raw biomass material.

According to at least one example embodiment, mixing of the first and second biomass-based streams results in a weight ratio of galactose to xylose in the fermentation media closer to 1 compared to the first stream weight ratio and/or the second stream weight ratio, whichever being based on the biomass-based stream having the highest xylose content.

Hereby, the mixing of the at least two biomass-based streams results in an improved weight ratio of galactose to xylose in the fermentation media.

According to at least one example embodiment, the galactose to xylose weight ratio is determined based on known composition of the first and second biomass-based streams and known mixing proportion or mixing ratio of the first and second biomass-based streams (based on weight, e.g. dry weight). According to at least one example embodiment, the galactose to xylose weight ratio is determined based on measurements of the first and second biomass-based streams and/or measurements of the fermentation media. Such measurements typically comprise direct or indirect measurements of the sugar content in the first and second biomass-based streams and/or the fermentation media, e.g. using HPLC.

According to at least one example embodiment, the determination of the galactose to xylose weight ratio in the fermentation media is performed in a continuous manner. Thus, the weight ratio of galactose to xylose may be measured in the fermentation media during the fermentation of the fermentation media, applicable e.g. during a continuous fermentation process. Additionally or alternatively, the adjustment of the proportion of the first and second biomass-based streams in order to adjust the galactose to xylose weight ratio is performed in a continuous manner. Thus, the adjustment of the proportion of the first and second biomass-based streams may be performed continuously in response to the determination of the galactose to xylose weight ratio in the fermentation media.

According to at least one example embodiment, the determination of the galactose to xylose weight ratio in the fermentation media is performed at the start of, or prior to, the fermentation of the fermentation media. Thus, such determination of galactose to xylose weight ratio may be referred to as an initial weight ratio of galactose to xylose in the fermentation media. Additionally or alternatively, the adjustment of the proportion of the first and second biomass-based streams in order to adjust the galactose to xylose weight ratio is performed in response to such determination of the of the galactose to xylose weight ratio in the fermentation media at the start of, or prior to, the fermentation of the fermentation media. This may e.g. be applicable in batch, or fed-batch fermentation processes.

According to at least one example embodiment, the fermentation media further comprises glucose. Glucose may repress the galactose metabolism. An abrupt exhaustion of glucose by a fast glucose fermentation rate may under anaerobic fermentation conditions result in a stuck fermentation as there is not enough stored energy (as ATP) in fast-growing cells for the initial necessary synthesis of galactose enzymes and cofactors to start galactose fermentation after glucose exhaustion. Another possibility is that both galactose and xylose fermentation proceeds at a lower rate than glucose, both due to limitations in the initial steps in the metabolic pathway. Moreover, as xylose does not repress galactose fermentation, and there seems to be an underutilized fermentation capacity in the cells in the later steps of the metabolism, it is possible that a true additive simultaneous fermentation can take place if both sugars are present.

It should be noted that the simultaneously fermentation of xylose and galactose implies that at least during a portion of the fermentation of the fermentation media, the xylose and galactose are simultaneously fermented. For example, during a majority of the fermentation process in which xylose and galactose are fermented, the xylose and galactose are fermented simultaneously. The step of fermenting the fermentation media is typically performed by means of fermentation microorganisms having a xylose isomerase, or alternatively, a xylose reductase and a xylitol dehydrogenase. Thus, the fermentation microorganisms have been adapted to ferment xylose in addition to hexoses.

According to at least one example embodiment, the xylose and galactose are co-fermented during the fermentation of the fermentation media.

It should be understood that when stating that the microbial fermentation process is substantial anaerobic, the fermentation of the fermentation media by means of fermentation microorganisms is carried out anaerobically, i.e. fermentation without the presence of oxygen, or at least in a process in which oxygen is not taking part in the fermentation. Stated differently, the substantial anaerobic fermentative process is a process without using oxygen as terminal electron acceptor. Throughout the application, the step of fermenting or fermentation may refer to such substantial anaerobic fermentation process.

Fermentation of a fermentation media may be defined as anaerobic metabolism of the sugars in the fermentation media. Thus, the fermentation microorganisms, such as yeast, metabolizes the sugars in the fermentation media in a fermentative manner even if oxygen is available, as oxygen is not taking part in the metabolism. According to at least one example embodiment, fermentation of a fermentation media implies extraction of energy from carbohydrates in the absence of oxygen participation. The absence of oxygen participation is related to that the fermentation microorganism (or yeast) does not use the oxygen even if it is present as long as there is glucose available, oxygen is thus not participating in the process.

According to at least one example embodiment, the substantial anaerobic microbial fermentation process is carried out in an oxygen reduced environment. The oxygen reduced environment is oxygen deficient. According to the present disclosure, "oxygen reduced environment" means that the amount of oxygen is reduced compared to the amount of oxygen present in air. In one embodiment, the oxygen concentration in the oxygen reduced environment is reduced with at least 50%, such as at least 75%, such as at least 80%, such as at least 85%, such as at least 90%, such as at least 96%, such as at least 97%, such as at least 98% or such as at least 99% compared to the oxygen concentration in air (in vol %). In one embodiment, about 1%, such as about 2%, such as about 5%, such as about 10%, or such as about 15% of oxygen is present in the oxygen reduced environment (oxygen concentration in vol %).

According to at least one example embodiment, the substantial anaerobic microbial fermentation process is a microaerobic microbial fermentation process, or an anaerobic microbial fermentation process.

According to at least one example embodiment, the fermenting is performed at an acetic acid concentration of between 0.1 and 50 g/L. For example, the lower concentration of the acetic acid during the fermentation is 0.1, or 0.5, or 1 g/L, while the higher concentration of acetic acid during the fermentation is 50, or 30, or 20, or 18, or 12 g/L. For example, the acetic acid concentration during the fermentation is between 0.5 and 12 g/L.

According to at least one example embodiment, the acetic acid concentration is referring to initial acetic acid concentration at the start of, or just prior to, the fermentation of the fermentation media.

According to at least one example embodiment, the acetic acid concentration is between 0.1 and 40 g/L, such as e.g. between 0.1 and 30 g/L or between 0.1 and 20 g/L, or between 0.1 and 18 g/L, or between 0.1 and 15 g/L, or between 0.1 and 12 g/L, or between 0.1 and 10 g/L, or between 0.1 and 8 g/L, or between 0.1 and 6 g/L, or between 0.1 and 4 g/L, or between 0.1 and 2 g/L.

According to at least one example embodiment, the acetic acid concentration is between 0.5 and 40 g/L, such as e.g. between 1 and 40 g/L or between 1.5 and 40 g/L, or between 3 and 40 g/L, or between 5 and 40 g/L, or between 8 and 40 g/L, or between 10 and 40 g/L, or between 12 and 40 g/L, or between 15 and 40 g/L, or between 18 and 40 g/L, or between 20 and 40 g/L.

According to at least one example embodiment, the acetic acid concentration is between 0.5 and 30 g/L, such as e.g. between 1 and 20 g/L or between 1.5 and 18 g/L, or between 3 and 15 g/L, or between 5 and 12 g/L, or between 8 and 10 g/L.

According to at least one example embodiment, the upper range in the interval of the acetic acid concentration is between 2 and 40, such as e.g. 40, 30, 20, 18, 15, 12, 10, 8, 6, 4, or 2 g/L. Additionality, or alternatively, the lower range in the interval of the acetic acid concentration is between 0.1 and 20, such as e.g. 0.1, 0.5, 1, 1.5, 3, 5, 8, 10, 12, 15, 18 or 20 g/L.

According to at least one example embodiment, the ratio of the initial concentration of the fermentation microorganisms and the initial concentration (g/L) of acetic acid in the fermentation media is between 0.1 and 1, or more preferably between 0.2 and 0.7.

Hereby, the amount of the fermentation microorganisms, such as yeast of the genus S. cerevisiae, may be adapted based on the amount of the acetic acid in the fermentation media. That is, the initial concentration of the acetic acid may be based on the initial concentration of the fermentation microorganisms. According to at least one example embodiment, the initial concentration (g/L) of the fermentation microorganisms to the initial concentration (g/L) of acetic acid in the fermentation media is 1:5 to 1:1.5.

According to at least one example embodiment, the ratio of the initial concentration (g/L) of the fermentation microorganisms and the initial concentration (g/L) of acetic acid in the fermentation media is between 0.3 and 0.7, or between 0.4 and 0.7, or between 0.5 and 0.7, or between 0.2 and 0.6, or between 0.2 and 0.5, or between 0.2 and 0.4, or between 0.3 and 0.6, or between 0.4 and 0.5.

According to at least one example embodiment, the fermenting comprises simultaneously fermenting the xylose and galactose at an improved fermentation rate compared to the average rate obtained by the corresponding individual fermentation of xylose and galactose. According to at least one example embodiment, the fermenting comprises simultaneously fermenting the xylose and galactose at a fermentation rate of at least the corresponding fermentation rate of galactose alone. As the fermentation rate of xylose alone is much lower than the fermentation rate of galactose alone, this means that the simultaneously fermentation of xylose and galactose improves the fermentation of xylose. That is, the fermentation rate of xylose is increased in the presence of, and in co-fermentation with(or simultaneously fermentation with) galactose compared to when xylose is fermented without the presence of galactose.

Hereby, the overall fermentation rate of the fermentation media is improved compared to fermenting the same fermentation media under the same conditions but for which just one of the sugars of xylose and galactose is present.

According to at least one example embodiment, the improved fermentation rate of the simultaneous fermentation of xylose and galactose is the same, or approximately the same, as the individual fermentation rate of galactose. Alternatively, the improved fermentation rate is the sum, or approximately the sum, of the individual fermentation rates of xylose and galactose under the same fermentation conditions.

According to at least one example embodiment, the fermenting comprises simultaneously fermenting the xylose and galactose with an improved acetic acid tolerance compared to corresponding individual fermentation of xylose and galactose.

Hereby, any negative impact originating from the acetic acid, typically at relatively high acetic acid concentrations, e.g. higher than 12 g/L or 20g/L, are less prominent than for corresponding individual fermentation of xylose and galactose. Thus, the simultaneous fermentation of xylose and galactose is improved compared to fermenting the same fermentation media under the same conditions but for which only one of the sugars xylose and galactose is present. In more general terms, the improved fermentation of simultaneously fermenting xylose and galactose results in an increased resistance to some fermentation inhibitors, for example relatively high acetic acid concentrations, such that the simultaneous fermentation of galactose and xylose supposedly improves fermentation at an inhibitor concentration compared to fermenting the same fermentation media under the same conditions but for which only one of the sugars of xylose and galactose is present.

According to at least one example embodiment, the target fermentation product is a monohydric alcohol, such as e.g. ethanol and all isomers of propanol and butanol with only one hydroxy group.

According to such embodiments, both the xylose and the galactose are fermented into the monohydric alcohol.

According to at least one example embodiment, the target fermentation product is not xylitol. That is, according to at least one example embodiment, xylitol is disclaimed from the target fermentation product.

According to at least one example embodiment, the initial concentration of galactose in the fermentation media is at least 1 or 2 g/L and the initial concentration of xylose in the fermentation media is at least 1 or 2 g/L, and/or wherein the ratio of initial galactose concentration (g/L) and initial xylose concentration (g/L) in the fermentation media is at least 0.1, such as at least 0.12, or at least 0.15, or at least 0.166.

According to at least one example embodiment, the initial amount of xylose relative to the total amount of fermentable sugars in the fermentation media is at least 2 wt% such as at least 5 wt%.

The fermentable sugars may e.g. be sugars which are fermentable by Saccharomyces cerevisiae (S. cerevisiae) or another variant of Saccharomyces. The initial amount of xylose relative to the total amount of fermentable sugars in the fermentation media may be at least 10 wt%, or at least 15 wt%, or at least 20 wt%.

According to at least one example embodiment, the initial amount of galactose relative to the total amount of fermentable sugars in the fermentation media is at least 2 wt% such as at least 5 wt%, or at least 10 wt%, or at least 15 wt%, or at least 20 wt%.

According to at least one example embodiment, the fermentation microorganisms is a yeast of the genus Saccharomyces or an engineered variant thereof.

As described above, such yeast is characterised by being able to ferment xylose (the yeast e.g. comprising a xylose isomerase, or alternatively, a xylose reductase and a xylitol dehydrogenase, both alternatives enabling the yeast to perform intreacellular conversion of xylose into xylulose). Stated differently, the yeast has been genetically engineered to be able to ferment one or more pentose sugars, such as xylose and may be referred to as a xylose fermenting engineered yeast. Thus, the yeast is configured to ferment xylose, and possibly other pentoses in addition to hexoses such as galactose and glucose. Hereby, the overall yield of the target fermentation product may be increased. According to at least one examples, the yeast is as any yeast strain belonging to the Saccharomyces genus (e.g. Saccharomyces cerevisiae, Saccharomyces bayanus, Saccharomyces uvarum). Yeast may refer to any organism belonging to the family Saccharomycetaceae, which include all the budding yeasts, e.g. also the Pichia genus.

According to at least one example embodiment, the biomass raw material is derived from hardwood, softwood, agricultural waste, algae or any mixture thereof.

Hereby, a mixture of different biomass materials can be used to reach the desired weight ratio of galactose to xylose in the fermentation media.

According to at least one example embodiment, the fermentation process is a continuous fermentation process, or is a fed-batch fermentation process.

Continuous, or fed-batch or semi-continuous fermentation, may be preferred over batch-wise fermentation, as the production of the target fermentation product may be improved (e.g. increased yield or with an increased fermentation rate) and/or simplified. Semi-continuous fermentation may also be referred to as repeated fed batch, e.g. a fed batch process in which half the content from the fermentation vessel is drained when the fermentation is finished, whereafter the feeding process is repeated. This can be repeated several times.

According to at least one example embodiment, the fermentation microorganisms do not comprise an inserted galactose inducible promoter regulating expression of genes involved in pentose metabolism.

For example, the fermentation microorganisms are free of an inserted galactose inducible promoter for pentose metabolism.

According to a third aspect of the present invention, use of xylose to improve galactose fermentation by means of fermentation microorganisms, such that the xylose and galactose are simultaneously fermented into the same target fermentation product, is provided.

The inventors have realized that the xylose may be used to promote galactose fermentation. For example, when galactose is co-fermented with glucose, the abrupt exhaustion of glucose requires an equally abrupt switch to galactose fermentation. Under anaerobic fermentation conditions, this may result in a stuck fermentation as there is not enough stored energy (as ATP) in the cells for the initial synthesis of enzymes and cofactors in the Leloir pathway to start galactose fermentation. This may be avoided by ensuring sufficient amounts of xylose to provide sufficient energy and time for initiating galactose fermentation.

According to at least one example embodiment, xylose is used to improved acetic acid tolerance compared to corresponding individual fermentation of xylose and galactose.

According to a fourth aspect of the present invention, use of galactose to improve xylose fermentation by means of fermentation microorganisms, such that the xylose and galactose are simultaneously fermented into the same target fermentation product, is provided.

Effects and features of the third and fourth aspects of the invention are largely analogous to those described above in connection with the first and second aspects of the invention. Embodiments mentioned in relation to the first and second aspects of the invention are largely compatible with the third and fourth aspects of the invention, in particular related to the target fermentation product and the fermentation microorganism. For example, the fermentation microorganism may have having a xylose isomerase, or alternatively, a xylose reductase and a xylitol dehydrogenase.

According to a fifth aspect of the present invention, a system for a microbial fermentation process involving fermentation of a fermentation media derived from biomass to a target fermentation product, is provided. The system comprises:

- a fermentation unit configured to ferment a fermentation media comprising at least xylose and galactose by means of fermentation microorganisms, such that the xylose and galactose are simultaneously fermented into the same target fermentation product.

Effects and features of this fifth aspect of the invention are largely analogous to those described above in connection with the first and second aspects of the invention. Embodiments mentioned in relation to the first and second aspects of the invention are largely compatible with the fifth aspect of the invention, of which some are exemplified below (typically without repeating the advantageous effects again). Thus, the system may be configured to enable fermentation performed at the presence of acetic acid, e.g. at a concentration of between 0.1 and 50 g/L, and the fermentation unit may be configured to achieve a substantially anaerobic microbial fermentation process.

According to at least one example embodiment, the system further comprises means for providing a first biomass-based stream and a second biomass-based stream different to the first biomass-based stream, the first and second biomass-based streams together comprising xylose and galactose. The means for providing different first and second biomass-based streams may e.g. comprise separate feeding units, separate transport piping, and separate containers for storing and/or hydrolysing the first and second biomass-based streams.

According to at least one example embodiment, the system further comprises means for mixing the first and second biomass-based streams. The means for mixing the first and second biomass-based streams may e.g. be a mixing device such as a screw feeder, a T-coupling or a mixing container.

According to at least one example embodiment, the system further comprises means for treating the mixed first and second biomass-based streams, or means for treating the first and second biomass-based streams separately prior to the mixing, to provide a fermentation media comprising xylose and galactose. The mixing of the first and second biomass-based streams results in a weight ratio of galactose to xylose in the fermentation media within a target range s described with reference to the second aspect of the invention, or result in a weight ratio of galactose to xylose in the fermentation media closer to the reference curve (or the area above the reference curve) compared to at least one of the first and second biomass-based streams, as described with reference to the first aspect of the invention. The means for treating the first and second biomass-based streams may e.g. comprise a pretreatment arrangement including e.g. an impregnation vessel for impregnating the first and second biomass-based streams with an acid, or SO2, and/or a reactor for treating the impregnated biomass at an elevated temperature and pressure. Alternatively or additionally, the means comprises a hydrolyzing unit, or a mixing device.

According to at least one example embodiment, the system comprises a control unit with program code means comprising instructions to perform at least some steps mentioned in relation to the first and/or second aspects of the invention. For example, the program code mans may comprise instructions of:

- providing a reference curve defined by a predetermined weight ratio of galactose to xylose as a function of acetic acid concentration present in the fermentation media;
- determining the galactose to xylose weight ratio in the fermentation media;

  - in response of determining that the galactose to xylose weight ratio in the fermentation media is below the reference curve, adjusting the proportion of the first and second biomass-based streams in order to adjust the galactose to xylose weight ratio to be above the reference curve. For example, the program code means comprises instructions to operate a valve in order to vary the mixing of the first and second biomass-based streams. Moreover, the program code means may comprise instructions for operating a determining device, or measuring device, configured to determine the galactose to xylose weight ratio in the fermentation media.

Alternatively, the program code mans may comprise instructions of determining the galactose to xylose weight ratio in the fermentation media, and in response of determining that the galactose to xylose weight ratio in the fermentation media is outside of the target range, adjusting the proportion of the first and second biomass-based streams in order to adjust the galactose to xylose weight ratio towards the target range.

Thus, the system may comprise means of controlling that a weight ratio of galactose to xylose of the fermentation media is within a target range, or that the weight ratio of galactose to xylose in the fermentation media is closer to the reference curve compared to at least one of the first and second biomass-based streams.

The means of controlling that the weight ratio of galactose to xylose of the fermentation media depends on where in the process of providing biomass derived raw material to prior to the fermentation, the mixing of the first and second biomass-based streams is carried out. The mixing means may e.g. comprise a mixing device for mixing the first and second biomass-based streams. The mixing device may be configured to mix of the first and second biomass-based streams prior to pretreating the biomass material, i.e. subjecting the biomass material to a pretreatment process, or the mixing device may be configured to mix the first and second biomass-based streams subsequent to the pretreatment process, and e.g. prior hydrolysis of the pretreated biomass, or the mixing device may be configured to mix the first and second biomass-based streams subsequent to the hydrolysis step, but prior to the fermentation. Moreover, in some embodiments, the mixing device is omitted, and the first and second biomass-based streams are directly fed to the fermentation unit.

According to at least one example embodiment, the system further comprises an acetic acid determination arrangement configured to determine the acetic acid concentration in the fermentation unit.

According to at least one example embodiment, the fermentation media is achieved as described in relation to the first or second aspects of the invention.

According to at least one example embodiment, the system further comprises a determining device, or a measuring device, configured to measure the xylose and galactose concentrations in the first and/or second biomass-based streams and/or in the fermentation media. For example, an HPLC system (high-performance liquid chromatography system) may be used to determine the concentration of various sugars in the fermentation unit, and/or the first and/or the second biomass-based streams. Such measuring device is particular advantageous for a continuous, a semi-continuous, or a fed-batch fermentation process.

According to at least one example embodiment, the system and the fermentation unit is configured to carry out the fermentation as a continuous, a semi-continuous, or a fed-batch fermentation process.

According to at least one example embodiment, the fermentation unit is a first fermentation unit and the system further comprises a second fermentation unit, the first and second fermentation units being configured to subsequently ferment the fermentation media. The fermentation unit(s) may be fermentation vessel(s). For example, the second fermentation unit may be arranged upstream of the first fermentation unit, and each one of the first and second fermentation units may according to an alternative embodiment be configured to ferment the fermentation media at different acetic acid concentrations. According to such embodiments, the system may comprise another fermentation unit being configured to ferment the fermentation media with an untampered acetic acid concentration, or at least in the absence of a set acetic acid concentration.

The system may comprise additional units and components known to those skilled in the art. For example, a pretreatment arranged for pretreating the biomass, a hydrolysis unit for hydrolyzing the pretreated biomass, and/or a separation unit arranged between the pretreatment arrangement and the hydrolysis unit, and/or between the hydrolysis unit and the fermentation unit. If not stated otherwise, any ratio of sugars, e.g. galactose to xylose, is based on weight (w/w), and may e.g. be derived by taking a ratio of the sugar concentrations (g/L), e.g. galactose concentration (g/L) to the xylose concentration (g/L). If not stated otherwise, any concentration is in the unit g/L.

## BRIEF DESCRIPTION OF THE DRAWINGS

The various aspects of the present invention, including its particular features and advantages, will be readily understood from the following detailed description and the accompanying drawings, in which:

Fig. 1 schematically illustrates an arrangement for treatment of biomass including control of a microbial fermentation process in accordance with example embodiments of the invention,

Fig. 2 schematically illustrates a system for a microbial fermentation process involving fermentation of a fermentation media from biomass to a target fermentation product in accordance with example embodiments of the invention,

Fig. 3 schematically illustrates the steps of a method for a microbial fermentation process in accordance with example embodiments of the invention,

Figs. 4-13, 14A, 14B and 14C are a graphs showing the progress of the fermentation in terms of $CO_2$ loss (in mg) over time (hours) of various series with measurement trials or samples, in accordance with example embodiments of the invention;

Figs. 15-16 are graphs of an exponential function representing galactose/xylose ratios in the fermentation media to obtain a benefit at various acetic acid concentrations, related to example embodiments of the invention;

Figs. 17-23 are graphs visualizing substrates and various possible results for mixing such substrates, related to example embodiments of the invention;

Figs. 24-27 show various galactose and xylose compositions for various substrates and the mixtures thereof, related to example embodiments of the invention; and

Figs. 28-29 show the pH dependence of the effective AcOH concentration and the effect of pH on the reference curve of Figs. 15-16.

## DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

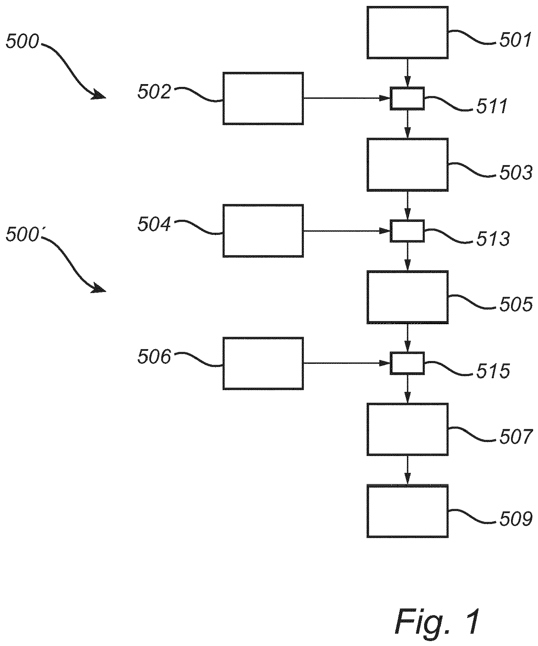

With reference to Fig. 1, the present disclosure provides an arrangement or system 500 for treatment of biomass comprising a supply unit 501 for supplying the biomass as a raw material, a pretreatment arrangement 503 for pre-treating the biomass, a hydrolysis unit 505 in which the pretreated biomass is subject to enzymatic hydrolysis by means of saccharification enzymes, the hydrolysis unit 505 being arranged downstream of and in fluid communication with the pretreatment arrangement 503, and comprises a fermentation unit 507, such as a fermentation vessel 507, arranged downstream of and in fluid communication with the hydrolysis unit 505. In the fermentation unit 507, the hydrolysate, or fermentation media, comprising fermentable sugars is fermented into a target chemical, e.g. ethanol, by means of fermentation microorganisms, such as a yeast (e.g. S. cerevisiae or an engineered variant thereof). The arrangement 500 may also comprise a product recovery unit 509, such as distillation or ion exchange chromatography, arranged downstream of and in fluid communication with the fermentation unit 507. As will be described in more detail in the following, the fermentation unit 507 typically comprises acetic acid, and may thus be configured to ferment a fermentation media comprising at least xylose and galactose by means of fermentation microorganisms having a xylose isomerase, or alternatively, a xylose reductase and a xylitol dehydrogenase, such that the xylose and galactose are simultaneously fermented into the same target fermentation product in the presence of such acetic acid concentration.

In Fig. 1, the fermentation unit 507 is comprised in an arrangement 500' for providing a fermentation media comprising at least xylose and galactose, and fermenting such fermentation media by means of fermentation microorganisms having a xylose isomerase, or alternatively, a xylose reductase and a xylitol dehydrogenase, such that the xylose and galactose are simultaneously fermented into the same target fermentation product. As mentioned above, the fermentation may be performed at the presence of an acetic acid, e.g. at a concentration of between 0.1 and 50.

In Fig. 1, the fermentability of the microbial fermentation process is improved by controlling a weight ratio of the specific sugars, in particular xylose and galactose, in the fermentation media. In more detail, the fermentation media in the fermentation unit 507 is achieved by providing a first biomass-based stream and a second biomass-based stream, and mixing the first and second biomass-based streams. The second biomass-based stream is different to the first biomass-based stream, and the first and second biomass-based streams together comprises at least the two fermentable sugars xylose and galactose. The mixing of the first and second biomass-based streams may be performed in various ways depending on where in the the process of from providing biomass raw material, e.g. by supply unit 501, to prior the fermentation process in the fermentation unit 507, the mixing of the first and second biomass-based streams is carried out.

In Fig. 1, three potentials process positions of the mixing are shown, of which only one is needed. A first mixing position is arranged downstream of the supply unit 501 and upstream of the pretreatment arrangement 503, in which the first biomass-based stream is provided by the supply unit 501 as a first raw material, and the second biomass-based stream is provided by a secondary supply unit 502 as a second raw material, different to the first raw material. For example, the first raw material may be hardwood comprising at least glucose and xylose, and the second raw material may be softwood or algae comprising at least galactose. It should be noted that the first raw material may comprise both xylose and galactose, and/or the second raw material may comprise both xylose and galactose, as long as the mixture of the biomass-based streams based on the first and second raw materials comprises both xylose and galactose. The first and second raw materials may be mixed in a first mixing device 511, the first mixing device 511 being e.g. a container or screw feeder. A second mixing position is arranged downstream of the pretreatment arrangement 503 and upstream of the hydrolysis unit 505, in which the first biomass-based stream is provided by the pretreatment arrangement 503 as a first pretreated biomass slurry, and the second biomass-based stream is provided by a secondary pretreatment arrangement 504 as a second pretreated biomass slurry. For example, the first pretreated biomass slurry may be derived from hardwood and comprising at least glucose and xylose, and the second pretreated biomass slurry may be derived from softwood or algae and comprising at least galactose. The first and second biomass slurries may be mixed in a second mixing device 513, the second mixing device 513 being e.g. a container or screw feeder. A third mixing position is arranged downstream of the hydrolysis unit 505 and upstream of the fermentation unit 507, in which the first biomass-based stream is provided by the hydrolysis unit 505 as a first hydrolysate, and the second biomass-based stream is provided by a secondary hydrolysis unit 506 as a second hydrolysate. For example, the first hydrolysate may be derived from hardwood and comprising at least glucose and xylose, and the second hydrolysate may be derived from softwood or algae and comprising at least galactose. The first and second hydrolysates may be mixed in a third mixing device 515, the third mixing device 515 being e.g. a container or screw feeder or a T-coupling.

According to at least one example embodiment, at least one of the first raw material and the second raw material comprises xylose and galactose. Thus, the first biomass-based stream comprises xylose and galactose at a first stream weight ratio, and/or the second biomass-based stream comprises xylose and galactose at a second stream weight ratio. Hereby, the mixing of the first and second biomass-based streams may result in a weight ratio of galactose to xylose in the fermentation media which is closer to a desired weight ratio compared to the first stream weight ratio and/or the second stream weight ratio. Thus, the mixing of the first and second biomass-based streams may result in more desired galactose to xylose weight ratio in the fermentation media, than prior the mixing. For example, the initial concentration of galactose in the fermentation media is at least 2 g/L and the initial concentration of xylose in the fermentation media is at least 2 g/L, and/or the ratio of initial galactose concentration (g/L) and initial xylose concentration (g/L) in the fermentation media is at least 0.1, such as at least 0.12, or at least 0.15, or at least 0.166. For example, the initial concentration of galactose in the first biomass-based stream prior to mixing is at least 1 g/L, and the initial concentration of xylose in the second biomass-based stream prior to mixing is at least 1 g/L.

By providing first and second biomass-based streams with known characteristics and content of xylose and galactose, and mixing the first and second biomass-based streams, the weight ratio of galactose to xylose in the fermentation media can be controlled. Thus, the first, second and/or third mixing devices 511, 513, 515 may be considered means of controlling the weight ratio of galactose to xylose of the fermentation media. Thus, the previously mentioned arrangement 500' may comprise the fermentation unit 507 and any one of the first, second and third mixing device 511, 513, 515. The fermentation media achieved by mixing the first and second biomass-based streams may be controlled with consideration to a reference curve as will be described in the following, or it may be achieved by mixing the first and second biomass-based streams with consideration to a target range of the weight ratio of galactose to xylose in the fermentation media.

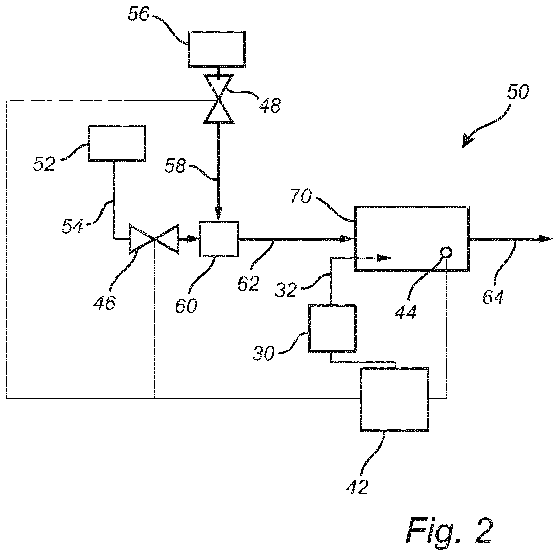

Fig. 2 schematically illustrates a system 50 for a microbial fermentation process involving fermentation of a fermentation media from biomass to a target fermentation product. The system 50 comprises a fermentation unit 70 configured to ferment a fermentation media 62 comprising at least xylose and galactose by means of fermentation microorganisms having a xylose isomerase, or alternatively, a xylose reductase and a xylitol dehydrogenase, such that the xylose and galactose are simultaneously fermented into the same target fermentation product, e.g. ethanol. The fermentation unit 70 comprises acetic acid e.g. at a concentration of between 0.1 and 50 g/L, and thus, the fermentation is configured to be carried out at the presence of acetic acid. The fermentation unit 70 may be adapted to be operated by a ratio of the initial concentration (g/L) of the fermentation microorganisms and the initial concentration (g/L) of acetic acid in the fermentation media of between 0.1 and 1, or between 0.2 and 0.7. Thus, acetic acid, originating from the first and/or second raw materials, or the process of treating the same in the pretreatment arrangement of Fig. 1, and the addition of fermentation microorganisms to the fermentation unit 70 may be controlled and set to a weight ratio of between 0.1 and 1.

As described with reference to Fig. 1, the system 50 may optionally comprise means for mixing two biomass-based streams used for achieving the fermentation media comprising at least xylose and galactose. Thus, the system 50 may comprise means 52 for providing a first biomass-based stream 54, the means 52 being e.g. a supply line from an upstream hydrolysis unit (as hydrolysis unit 505 of Fig. 1) or a moisture adjusting unit configured to intentionally increase or decrease the concentration of sugars in the first biomass-based stream 54. The system further comprises means 56 for providing a second biomass-based stream 58, the means 56 being e.g. a supply line from an upstream hydrolysis unit (as secondary hydrolysis unit 506 of Fig. 1) or a moisture adjusting unit configured to intentionally increase or decrease the concentration of sugars in the second biomass-based stream 58. The first and the second biomass-based streams 54, 58 together comprises at least two fermentable sugars being xylose and galactose. Moreover, the system 50 comprises a mixing device 60 configured to mix the first and the second biomass-based streams 54, 58 to provide a fermentation media 62, wherein the fermentation unit 70 is configured to ferment the fermentation media 62.

The system 50, and the fermentation unit 70, in Fig. 2 are configured to continuously ferment the fermentation media 62 by co-fermenting the fermentable sugars, at least simultaneous fermentation of xylose and galactose, by means of fermentation microorganisms of e.g. yeast, to the same target fermentation product 64. Moreover, the system 50 comprises a determining device 42 configured to determine the galactose to xylose weight ratio in the fermentation media 62 prior to, or in, the fermentation unit 70. The determining device 42 may e.g. determine the galactose to xylose weight ratio in the fermentation media 62 by known data of the xylose and galactose content in the first and second biomass-based streams 54, 58 e.g. by knowing the conversion/availability thereof from the first and second raw material. Alternatively, and as shown in Fig 2, the determining device 42 may comprise a measuring probe 44, or sample collector 44, being in direct contact with the fermentation media (or fermentation slurry) in the fermentation unit 70 for a measurement of the xylose and galactose concentrations. Such measurement may e.g. be performed by a HPLC (high-performance liquid chromatography.

The system 50 may further comprise a control unit, here exemplified as being comprised in the determining device 42. The control unit may comprise program code means comprising instructions to perform at least some of the following steps:

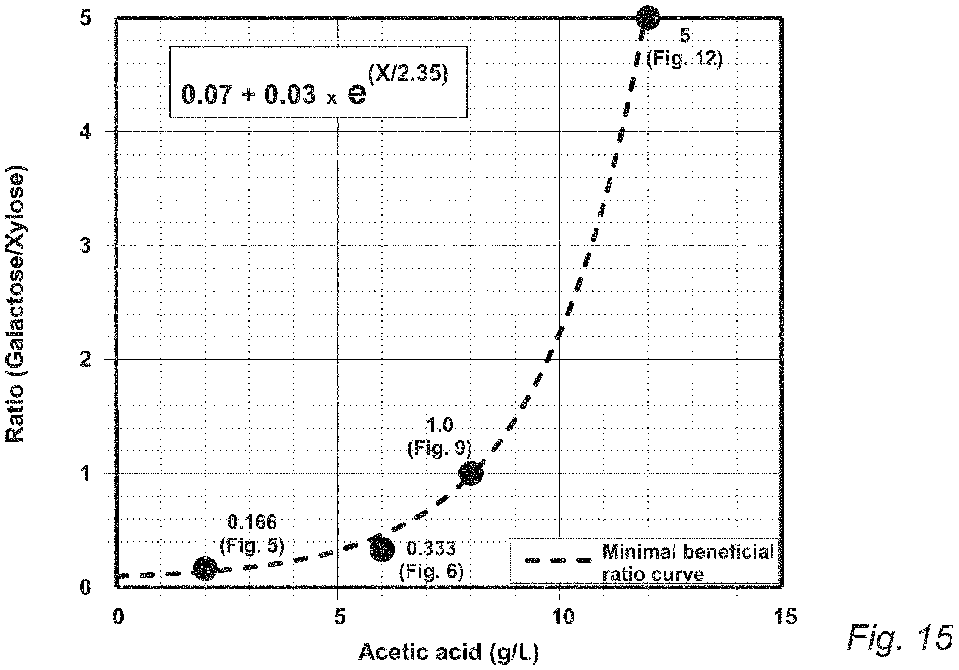

- providing a reference curve defined by a predetermined weight ratio of galactose to xylose as a function of acetic acid concentration present in the fermentation media;
- determining the galactose to xylose weight ratio in the fermentation media;
- in response of determining that the galactose to xylose weight ratio in the fermentation media is below the reference curve, adjusting the proportion of the first and second biomass-based streams in order to adjust the galactose to xylose weight ratio to be above the reference curve. The reference curve is further described with reference to Fig. 15 below.

Alternatively, the program code mans may comprise instructions of determining the galactose to xylose weight ratio in the fermentation media, and in response of determining that the galactose to xylose weight ratio in the fermentation media is outside of a target range, adjusting the proportion of the first and second biomass-based streams in order to adjust the galactose to xylose weight ratio towards the target range.

Thus, in response of determining that the galactose to xylose weight ratio in the fermentation media 62 is below, or offset compared to, the reference curve, or is outside of the target range, the system 50 may comprise means 46, 48 for adjusting the proportion of the first and second biomass-based streams 54, 58. Hereby, in order to adjust the galactose to xylose weight ratio towards, or above, the reference curve or target range, adaptation of the amount of fermentable sugars of xylose and galactose in the fermentation media 62 may be achieved by adapting the first and/or the second biomass-based streams 54, 58. This may e.g. be achieved by that the control unit of the determination device 42 controls a first valve 46 varying the amount of the first biomass-based stream 54 to the mixing device 60 and/or a second valve 48 varying the amount of the second biomass-based stream 58 to the mixing device 60. Alternatively, the determination device 42 may control the actual mixing in the mixing device 60. It should be understood that when adapting the amount of fermentable sugars of xylose and galactose in the fermentation media 62 by adapting the first and/or the second biomass-based streams 54, 58, the weight ratio of galactose to xylose is typically known in a predetermined manner, and can thus be kept within a desired range. Thus, a means of controlling the weight ratio of galactose to xylose of the fermentation media 62 is provided by the first and second valves 46, 48 and the provision of mixing a first and a second biomass-based streams together comprising xylose and galactose in the mixing device 60.

According to at least one example embodiment, the determination device 42 may be configured to automatically adapt the weight ratio of galactose to xylose towards, or above, the reference curve, or towards the target range of the galactose to xylose weight ratio in the fermentation media as previously described, in response to the determined sugar concentrations of xylose and galactose. Thus, the first and/or the second biomass-based streams 54, 58, may be directly controlled and adapted via the first and second valves 46, 48 to adapt the weight ratio of galactose to xylose. For such embodiments, the mixing device 60 may be omitted, and the first and second biomass-based streams 54, 58 directly fed to the fermentation unit 70.

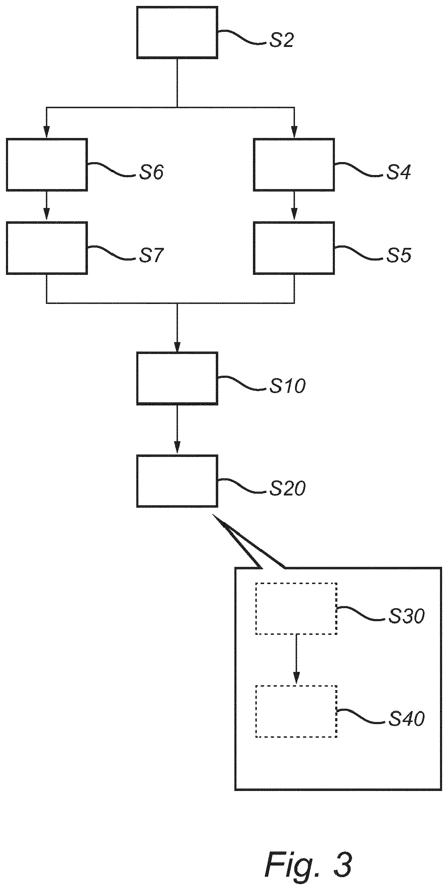

The invention will now be described with reference to the flow chart of Fig. 3 which e.g. includes the operation of the system 50 of Fig. 2. The flow chart of Fig. 3 discloses the steps of a method for a microbial fermentation process involving fermentation of a fermentation media from biomass to a target fermentation product. The microbial fermentation process may be substantially anaerobic.

In a step S10, a fermentation media comprising at least xylose and galactose is provided. The initial concentration of galactose in the fermentation media is e.g. at least 2 g/L and the initial concentration of xylose in the fermentation media is e.g. at least 2 g/L. Alternatively, the ratio of initial galactose concentration (g/L) and initial xylose concentration (g/L) in the fermentation media is e.g. at least 0.1, such as at least 0.12 or at least 0.15, or at least 0.166.

In a step S20, the fermentation media is fermented by means of fermentation microorganisms having a xylose isomerase, or alternatively, a xylose reductase and a xylitol dehydrogenase, such that the xylose and galactose are simultaneously fermented into the same target fermentation product. The fermenting may be performed at an acetic acid concentration of e.g. between 0.1 and 50 g/L. The fermentation product is typically an alcohol, such as e.g. ethanol.

This may e.g. be carried out by a fermentation unit (as fermentation unit 507 of Fig. 1). The fermentation microorganisms having a xylose isomerase, or alternatively, a xylose reductase and a xylitol dehydrogenase is e.g. a yeast or a xylose-engineered yeast, i.e. a yeast with the capability of fermenting xylose. Preferably, the step S20 of fermenting is performed in a continuous, or semi-continuous, manner in a fermentation unit.

According to at least one example embodiment, the presence (e.g. by addition or recirculation) of fermentation microorganisms and the presence of acetic acid during the step S20 of fermentation are controlled. Hereby, the ratio of the initial concentration (g/L) of the fermentation microorganisms and the initial concentration (g/L) of acetic acid in the fermentation media can be set to be between 0.1 and 1, or 0.2 to 0.7. The step S20 of fermentation typically comprises simultaneously fermenting the xylose and galactose at an improved fermentation rate compared to the average rate obtained by the corresponding individual fermentation of xylose and galactose. Hereby, at least an improved acetic acid tolerance compared to corresponding individual fermentation of xylose and galactose is provided.

The step S10 of providing the fermentation media may be preceded by one or several steps of achieved the fermentation media. For example, in a step S2, a first biomass-based stream and a second biomass-based stream different to the first biomass-based stream are provided. The first and second biomass-based streams together comprises xylose and galactose. The first and second biomass-based streams may be derived from hardwood, softwood, agricultural waste, algae or any mixture thereof. According to one example embodiment, one of the first and second biomass-based streams comprises at least xylose (e.g. derived from hardwood), and the other one of the first and second biomass-based streams comprises at least galactose (e.g. derived from softwood, such as spruce or pine, or algae). Additionality, at least one of the first and second biomass-based streams comprises glucose. Moreover, the first biomass-based stream may comprise xylose and galactose at a first stream weight ratio, and/or the second biomass-based stream may comprise xylose and galactose at a second stream weight ratio. For example, the initial concentration of galactose in the first biomass-based stream prior to mixing is at least 1 g/L, and the initial concentration of xylose in the second biomass-based stream prior to mixing is at least 1 g/L.

In a step S4, the first and second biomass-based streams are mixed. For example, the first and second biomass-based streams are mixed to reach a weight ratio of galactose to xylose within a target range. For example, the weight ratio of galactose to xylose in the fermentation media within the target range is closer to a desired weight ratio compared to the first stream weight ratio and/or the second stream weight ratio. Alternatively, the first and second biomass-based streams are mixed to reach a weight ratio of galactose to xylose in the fermentation media closer to the reference curve (or the area above the reference curve) compared to at least one of the first and second biomass-based streams.

In a step S5, the mixed first and second biomass-based streams are treated to provide a fermentation media comprising xylose and galactose.

In an alternative embodiment, the steps S4 and S5 are swopped so that each one of the separate first and second biomass-based streams are treated, shown as an alternative step S6, prior to a step of mixing the first and second biomass-based streams, shown as an alternative step S7.

Treating the mixed first and second biomass-based streams, or the separate first and second biomass-based streams may include at least one of the following actions: pretreating (e.g. in a pretreatment arrangement 503 described with reference to Fig. 1), hydrolyzing (e.g. in a hydrolyzing unit 505 described with reference to Fig. 1), addition of nutrients, adjustment of pH. For the separate pre-treatment and hydrolysis of the first and second biomass-based streams, treating may simply be referred to as mixing and/or transporting and/or temperature controlling the first and second biomass-based streams.

In either way, the result of the steps of treating and mixing, S4, S5, S6, S7, is to provide a fermentation media comprising xylose and galactose, with a weight ratio of galactose to xylose in the fermentation media within a target range, or a weight ratio of galactose to xylose closer to the reference curve (or the area above the reference curve) compared to at least one of the first and second biomass-based streams. The weight ratio of galactose to xylose in the fermentation media is different to the weight ratio of galactose to xylose in the separate first and/or second biomass-based streams. The amount of xylose in the fermentation media may be between 5 and 65 wt%, and the amount of galactose in the fermentation media may be between 1 and 65 wt%, as compared to the total amount of fermentable sugars in the fermentation media.

According to at least one example embodiment, the target range is a predefined target range, the predefined target range being based on an improved fermentation (with regards to fermentation time, fermentation rate and/or yield of the target fermentation product) compared to fermenting the same fermentation media under the same conditions but for which one of the sugars of xylose and galactose being removed, and/or for a fermentation media in which xylose and galactose are fermented subsequently and not simultaneously. Thus, by providing a fermentation media with a weight ratio of galactose to xylose within a target range, the fermentation may be improved. The target range may e.g. extend from 0.166 to 3.0. Such target range may be based on the respective concentrations of xylose and galactose.

In a step S30, which e.g. may be performed in parallel to the step S20 of fermentation, the galactose to xylose weight ratio in the fermentation media is determined. This may e.g. be achieved by the determination device 42 described with reference to Fig. 2.

In a subsequent step S40, and in response of determining that the galactose to xylose weight ratio in the fermentation media is outside of the target range, or is below, or offset compared to, the reference curve, the proportion or weight ratio of the first and second biomass-based streams are adjusted in order to adjust the galactose to xylose weight ratio in the fermentation media. This may e.g. be achieved by the determination device 42, and the first and second valves 46, 48 described with reference to Fig. 2.

The system 50 in Fig. 2 is configured to carry out the fermentation as a continuous fermentation process in the fermentation unit 70, but it may as well be configured as a batch fermentation process in which the fermentation media is fermented batch-wise. For embodiments in which the method is automated and adapted for continuous operation, the method may, alternatively or in addition to the target range of the weight ratio of galactose to xylose, comprise a step of collecting and monitoring various process parameters in a continuous or semi-continuous manner and a step of automatically adjusting the fermenting process in response to the monitored process parameters. If not stated otherwise, any weight ratio, or ratio of sugars, e.g. galactose to xylose, is based on weight (w/w), and may e.g. be derived by taking a ratio of the sugar concentrations (g/L), e.g. galactose concentration (g/L) to the xylose concentration (g/L). If not stated otherwise, any concentration is in the unit g/L.

## EXAMPLES

Co-fermentation of different fermentation media including sugar mixtures of various amounts of glucose, xylose and galactose in mineral minimal medium with B vitamins added, essentially as devised by Verduyn et al, 1992 (Verduyn C, Postma E, Scheffers WA, Van Dijken JP (1992). Effect of benzoic acid on metabolic fluxes in yeasts: a continuous-culture study on the regulation of respiration and alcoholic fermentation. Yeast 1992;8(7):501-17.), except that 3 g/L urea was used as nitrogen source instead of ammonia, in order to stabilize the pH value throughout the fermentation around the start pH value, in all examples adjusted to 5.5. Fermentations were performed batch-wise with 20 or 25 ml cultures in 50 ml closed bottles with a canula mounted to allow escape of the generated $CO_2$ Acetic acid was added as sodium/potassium acetate adjusted to pH 5.5 as well. The fermentation progressions were followed by monitoring $CO_2$ loss as milligram weight loss of the bottles at intervals during the experiments.

Different measurements were carried out for the sugar mixture with various amount of acetic acid. For all measurement trials or samples, a xylose fermenting engineered S. cerevisiae, commercially available as active dry yeast, was used after rehydration for 45-60 minutes as recommended by the manufacturer, using an inoculation amount as indicated in the examples, a temperature of 30 °C, and shaking of the cultures on an orbital shaker set at 140 rpm. For the yeast inoculation amount, reference is always to the dry weight (e.g. g dw / L). In the examples below, each sample or substrate may thus represent the first or the second biomass-based streams, or the fermentation media generated by mixing the first and the second biomass-based streams. Stated differently, mixing the first and the second biomass-based streams may correspond to the samples or substrates mentioned in the examples.

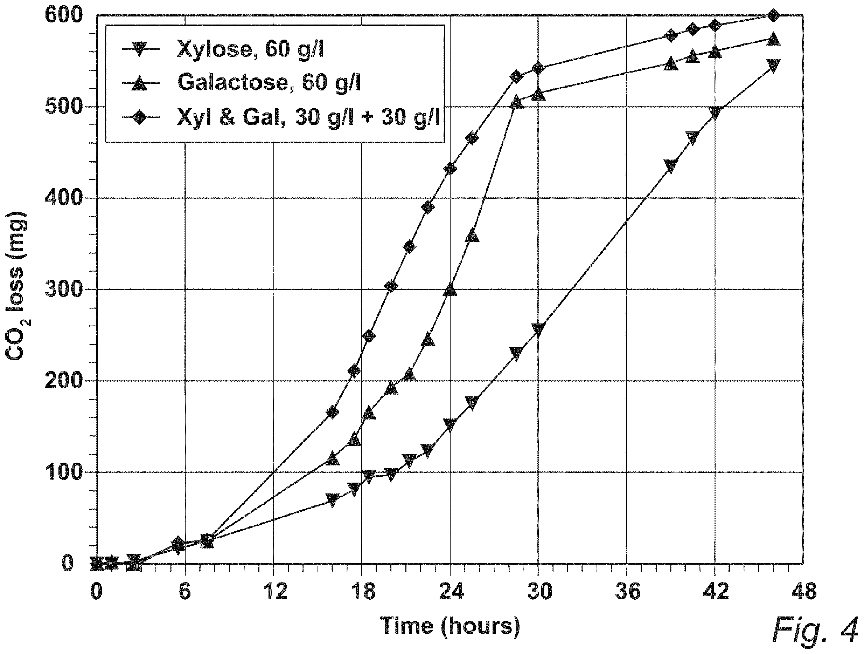

A first series of three measurement trials or samples is presented in the graph of Fig. 4 showing the progress of the fermentation in terms of $CO_2$ loss (in mg) over time (hours). For each of the samples, an initial yeast inoculation amount of 1 g/L was used at a relatively low or minimal amount of acetic acid at 0.1 g/L. In a first sample only xylose at an initial concentration of 60 g/L was fermented (referred to as fermentation of xylose only), in a second sample only galactose at an initial concentration of 60 g/L was fermented (referred to as fermentation of galactose only), and in a third sample xylose and galactose were co-fermented at a respective initial concentration of 30 g/L (referred to as co-fermentation of xylose and galactose).

It is clear from Fig. 4 that the third sample with co-fermentation of xylose and galactose is in total fermented faster than the first and second samples with fermentation of xylose only and galactose only. In fact, the third sample with co-fermentation of xylose and galactose indicates an additive fermentation effect, which is close to the sum of the individual fermentation of xylose only and galactose only, at least up to the time where galactose only sample is halfway through consumption of the sugar at 24 hours. At 24 hours of fermentation, the weight loss for the first sample with fermentation of xylose only is approximately 150 mg CO2, the weight loss for the second sample with fermentation of galactose only is approximately 300 mg CO2, and the weight loss for the third sample with co-fermentation of xylose and galactose is approximately 430 mg CO2, indicating that the fermentation rate of the co-fermentation of xylose and galactose up to this point is close to the sum of the fermentation rates of the individual fermentation of xylose only and galactose only. Thus, there is a clear advantage of co-fermenting xylose and galactose as compared to the fermentation of galactose only or xylose only. It is possible to add galactose to a xylose containing media up to two times the xylose concentration while maintaining the same total fermentation time, or to add xylose to a galactose containing feed in a proportion up to half the galactose concentration while maintaining the same total fermentation time.

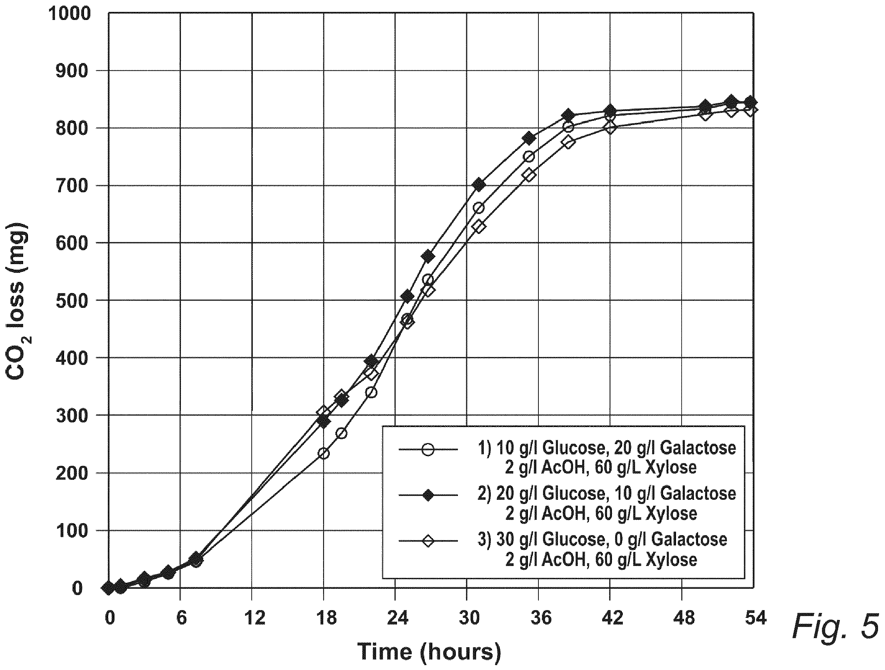

A second series of three measurement trials or samples is presented in the graph of Fig. 5 showing the progress of the fermentation in terms of CO2 loss (in mg) over time (hours). For each of the samples, an initial yeast inoculation amount of 1 g/L was used at a relatively low amount of acetic acid at 2 g/L. In sample 3, 30 g/L glucose was co-fermented with 60 g/L xylose. In sample 2, 10 g/L of the glucose has been replaced by galactose, maintaining everything else equal, and in sample 1, 20 g/L glucose has been replaced by galactose, everything else equal.

It is clear from Fig. 5 that exchanging glucose with galactose for a co-fermentation of glucose and xylose improves the fermentability of the xylose after a point in time when glucose has been exhausted or substantially exhausted. In Fig. 5, sample 3 without galactose appears to have the fastest fermentation in the beginning, and the two galactose containing samples are both a bit slower. This however is reversed after 22-24 hours, as in the last part of the fermentation, where the xylose is fermented, the overall fermentation rate is higher in sample 1 and 2, showing that the exchange of some glucose with galactose has increased the xylose fermentation rate. In other words, the xylose fermentation rate can be improved by addition of galactose.

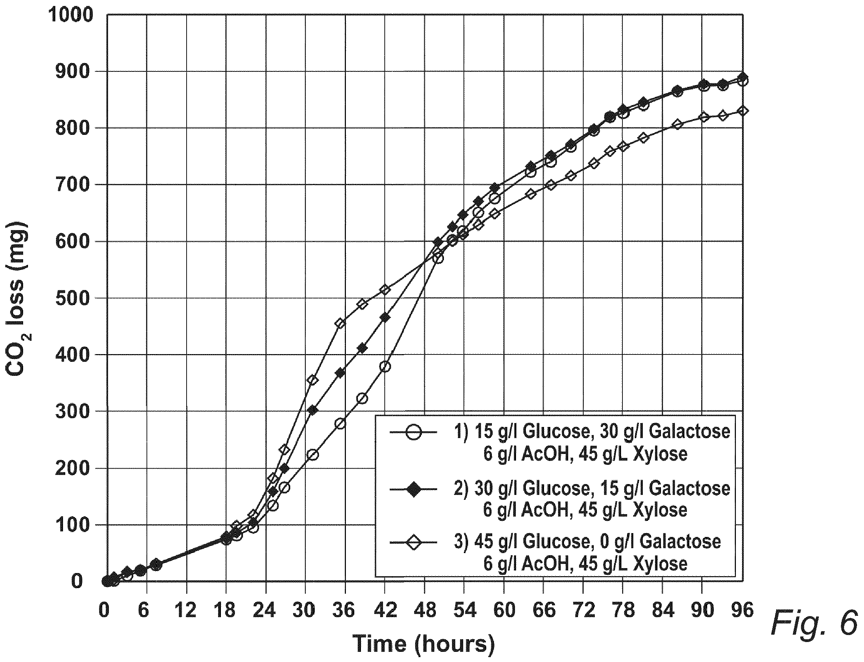

A third series of three measurement trials or samples is presented in the graph of Fig. 6. The example demonstrates the same as was described in fig. 5, this time in the presence of 6 g/L acetic acid and using a yeast inoculation amount of 1 g DW yeast/L, showing the progress of the fermentation in terms of $CO_2$ loss (in mg) over time (hours). In sample 1 xylose was co-fermented with galactose and glucose at initial concentrations 45 g/L xylose, 30 g/L galactose and 15 g/L glucose (referred to as co-fermentation of xylose and galactose at 45 g/L and 30 g/L), in sample 2 xylose was co-fermented with galactose and glucose at initial concentrations 45 g/L xylose, 15 g/L galactose and 30 g/L glucose (referred to as co-fermentation of xylose and galactose at 45 g/L and 15 g/L), and in sample 3 xylose was co-fermented with glucose at initial concentrations 45 g/L xylose and 45 g/L glucose (referred to as co-fermentation of xylose and glucose at 45 g/L and 45g/L.

It is clear from Fig. 6 that exchanging glucose with galactose for a co-fermentation of glucose and xylose improves the fermentability of the xylose after a point in time when glucose has been exhausted and/or substantially exhausted, as was also shown in Fig. 5. In Fig. 6, sample 3 with co-fermentation of xylose and glucose the fermentation rate declines sharply at approx. 35 hours (i.e. an elbow or bent of the curve is clearly shown at 35 hours), whereas sample 1 and sample 2 comprising galactose maintains a relatively high fermentation rate and exceeds sample 1 with co-fermentation of xylose and glucose at approximately 48 hours. Thus, during the latter part of the fermentation process (from 48 hours and forwards), sample 1 and sample 2 with co-fermentation of xylose and galactose at 15 and 30 g/L catches up with, and passes, sample 3 with co-fermentation of xylose and glucose. At the time where half of the fermentable sugars has been fermented, approx. at 450 mg $CO_2$ loss, sample 3 with co-fermentation of xylose and glucose is about 6 hours ahead of sample 2 co-fermentation of xylose and galactose 15 g/L, and 8-9 hours ahead of sample 1 with co-fermentation of xylose and galactose 30 g/L. However, at 700 mg $CO_2$ loss, sample 1 and sample 2 comprising galactose are between 5 and 8 hours ahead of sample 3 with co-fermentation of xylose and glucose only, demonstrating that galactose addition may be used for enhancement of the xylose fermentation, at least up to an acetic acid level of 6 g/l.

Thus, compared to the results on Fig. 5, the difference between the samples comprising galactose (first and second samples) compared to the samples comprising xylose and glucose only, is larger in Fig. 6. Also, by comparing to the results of Fig. 5, it is clear that a higher acetic acid content requires a larger amount of galactose relative to xylose (i.e. a higher galactose/xylose weight ratio).

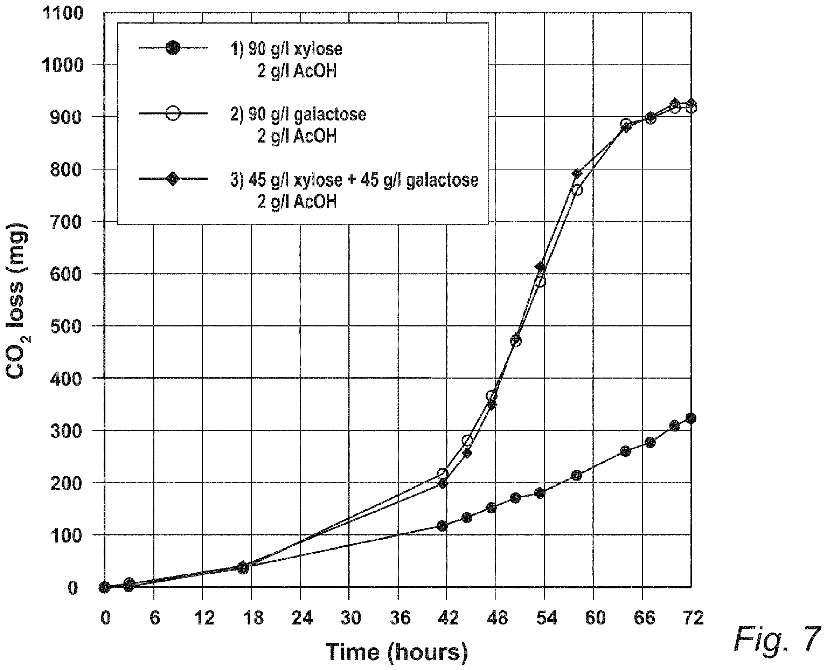

A fourth, fifth and sixth series of three corresponding measurement trials or samples are presented in the graphs of Figs. 7-9 showing the progress of the fermentation in terms of CO2 loss (in mg) over time (hours). For each of the samples, an initial yeast inoculation amount of 1 g/L was used at varying amount of acetic acid from 2 g/L (fourth series), to 6 g/L (fifth series) and 8 g/L (sixth series). In respective samples 1, xylose only was fermented at an initial concentration of 90 g/L (referred to as fermentation of xylose only), in respective samples 2, galactose only was fermented at an initial concentration of 90 g/L (referred to as fermentation of galactose only), and in respective samples 3, xylose was co-fermented with galactose at initial concentrations 45 g/L xylose and 45 g/L galactose (referred to as co-fermentation of xylose and galactose).

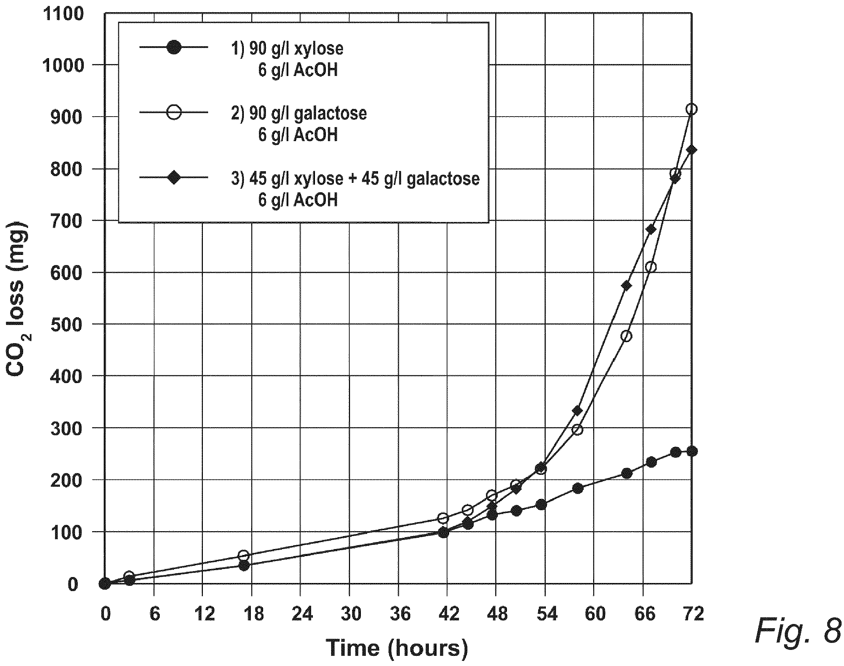

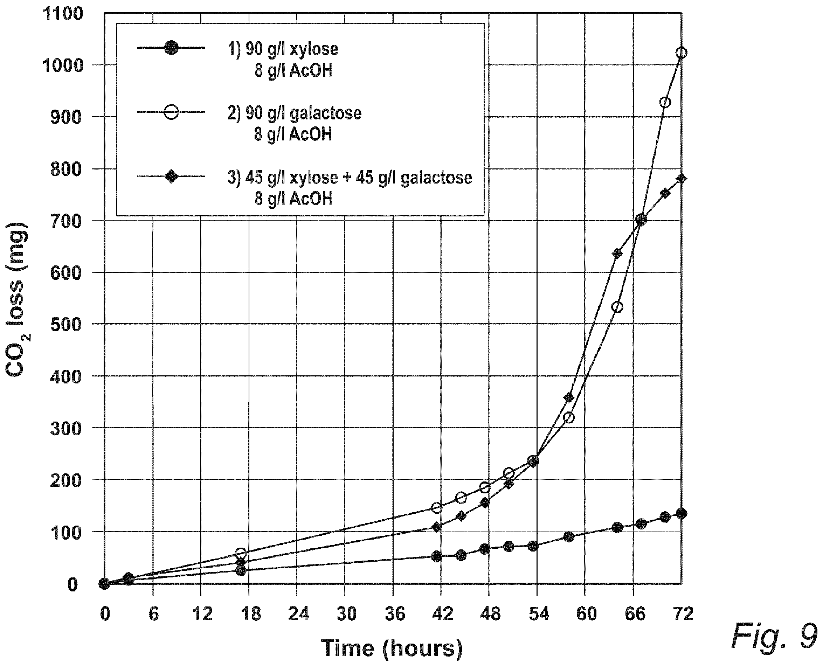

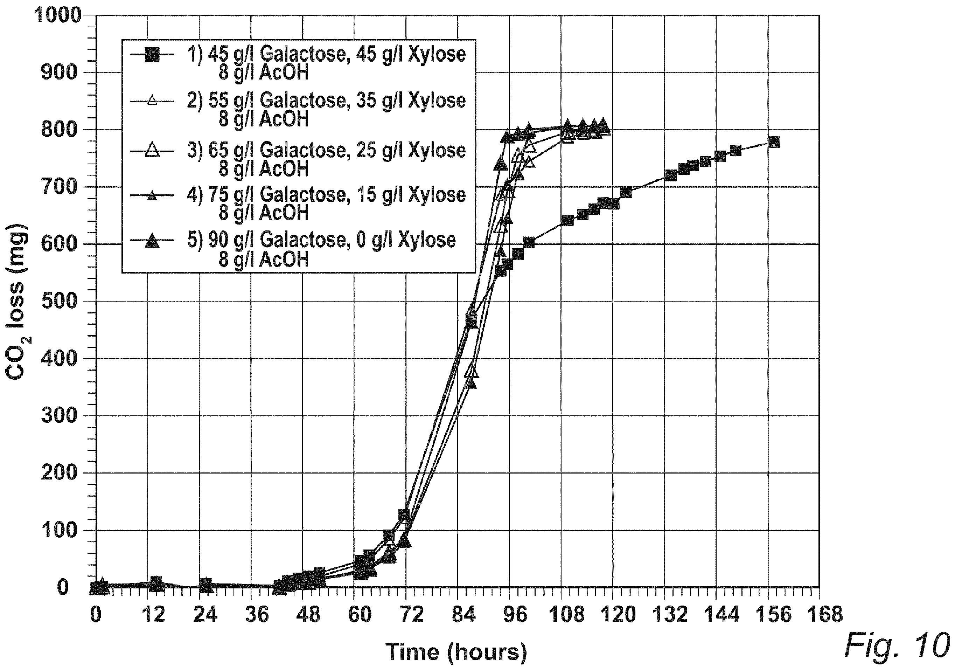

It is clear from Fig. 7 that the sample 2 and sample 3, showing fermentation of galactose only and co-fermentation of equal amounts of xylose and galactose at same total sugar amount, are fermented at an almost identical fermentation rate over the total fermentation time, the mixture being marginally faster between 42 hours and 58 hours. Sample 1 fermenting 90 g/L xylose has consumed less than 1/3 of the total sugar at the time where samples 2 and 3 have consumed all the sugar. Thus, the fermentation rate of xylose can be greatly improved when co-fermented with galactose instead of fermented isolated. From Fig. 8, it can be derived that by increasing the acetic acid concentration up to 6 g/L, the overall fermentation becomes a bit slower. Still, looking at the time span from 44 hours to 66 hours, the co-fermentation of xylose and galactose is faster than the fermentation of galactose only. But in contrast to sample 2 in Fig. 7, the relative rate of the co-fermentation of xylose and galactose compared to the fermentation of galactose only decrease at or after 66 hours or after 700 mg $CO_2$ loss. Fig. 9 shows the similar samples at 8 g/L acetic acid concentration. The increase in acetic acid concentration from 6 to 8 g/L does not change the fact that sample 2 with the co-fermentation of xylose and galactose is faster in part of the fermentation process, from 42 hours to 64 hours, at which point a sharp decline in rate is seen. This behaviour can be explained by assuming that the relative fermentation of galactose and xylose in the simultaneous fermentation changes with higher acetic acid concentration. As seen for sample 2 in Fig. 8 and in Fig. 9, it runs out of galactose earlier at higher acetic acid concentration. This is confirmed by a seventh series of five measurement trials of samples that is presented in the graph of Fig. 10 showing the progress of the fermentation in terms of

$CO_2$ loss (in mg) over time (hours). For each of the samples, an initial yeast inoculation amount of 1 g/L was used at a moderate to relatively high amount of acetic acid at 8 g/L. In sample 1 xylose was co-fermented with galactose at initial concentrations 45 g/L xylose and 45 g/L galactose (referred to as co-fermentation of xylose and galactose at a galactose/xylose weight ratio of 1), in sample 2 xylose was co-fermented with galactose at initial concentrations 35 g/L xylose and 55 g/L galactose (referred to as co-fermentation of xylose and galactose at a galactose/xylose weight ratio of 1.67), in sample 3 xylose was co-fermented with galactose at initial concentrations 25 g/L xylose and 65 g/L galactose (referred to as co-fermentation of xylose and galactose at a galactose/xylose weight ratio of 2.6), in sample 4 xylose was co-fermented with galactose at initial concentrations 15 g/L xylose and 75 g/L galactose (referred to as co-fermentation of xylose and galactose at a galactose/xylose weight ratio of 5), in sample 5 galactose only was fermented at an initial concentration of 90 g/L (referred to as fermentation of galactose only). The examples in Fig. 10 thus show samples with increasing galactose/xylose ratios that are tested compared to the one-to-one weight ratio used in Figs. 7-9.

It is clear from Fig. 10 that at acetic acid concentrations of 8 g/L, a higher galactose/xylose weight ratio is needed to obtain the positive effect of galactose supported xylose fermentation throughout the xylose fermentation. Samples 2-5 show a similar fermentation rate over the fermentation time, while sample 1 with co-fermentation of xylose and galactose at a galactose/xylose weight ratio of 1 is fermented slower and is levelling of already at approx. 90 hours. The fermentation at higher rate to completion and not just through part of the fermentation is shown obtained already by increasing the galactose xylose weight ratio from 45/45 to 55/35 at 8 g/L acetic acid concentration.

Thus, to obtain the benefit from the galactose supported xylose fermentation throughout the fermentation at acetic acid concentration up to or of 8 g/L, the galactose/xylose weight ratio should be at or above 1,67 such as e.g. between 1.67 and 5, or such as between 1.67 and 5. Thus, the co-fermentation of xylose and galactose can be greatly improved at a galactose/xylose weight ratio above 1, such as e.g. between 1 and 5, or above 1.67, such as between 1.67 and 5, at least up to an acetic acid level of 8 g/L.

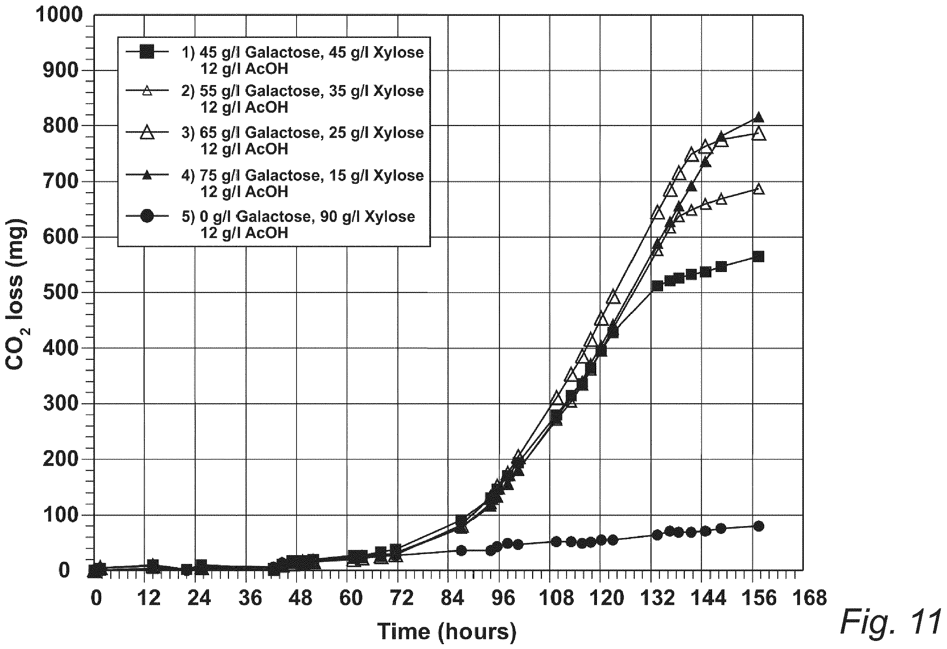

An eighth series of five measurement trials or samples, essentially similar to Fig. 10 but with further raised acetic acid content, is presented in Fig. 11 showing the progress of the fermentation in terms of $CO_2$ loss (in mg) over time (hours). For each of the samples, an initial yeast inoculation amount of 1 g/L was used at a relatively high concentration of acetic acid at 12 g/L. In sample 1 xylose was co-fermented with galactose at initial concentrations 45 g/L xylose and 45 g/L galactose (referred to as co-fermentation of xylose and galactose at a galactose/xylose weight ratio of 1), in sample 2 xylose was co-fermented with galactose at initial concentrations 35 g/L xylose and 55 g/L galactose (referred to as co-fermentation of xylose and galactose at a galactose/xylose weight ratio of 1.67), in sample 3 xylose was co-fermented with galactose at initial concentrations 25 g/L xylose and 65 g/L galactose (referred to as co-fermentation of xylose and galactose at a galactose/xylose weight ratio of 2.6), in sample 4 xylose was co-fermented with galactose at initial concentrations 15 g/L xylose and 75 g/L galactose (referred to as co-fermentation of xylose and galactose at a galactose/xylose weight ratio of 5), in sample 5 xylose only was fermented at an initial concentration of 90 g/L (referred to as fermentation of xylose only). Thus, samples 1-4 of the eighth series corresponds to samples 1-4 of the seventh series, but with a higher acetic acid concentration.

It is clear from Fig. 11 that the same tendency as could be seen in Fig. 10 is present also at this relatively high acetic acid concentration of 12 g/L, i.e. that a higher galactose/xylose weight ratio is needed to obtain the optimal positive effect of galactose supported xylose fermentation at a higher acetic acid concentration throughout the fermentation. The samples 3 and 4 show a similar fermentation rate over the fermentation time with sample 3 levelling of earlier than sample 4, while sample 1 with co-fermentation of xylose and galactose at a galactose/xylose weight ratio of 1, and sample 2 with co-fermentation of xylose and galactose at a galactose/xylose weight ratio of 1.67, are fermented slower towards the end of the fermentation and is levelling of already at approx. 122 hours and 138 hours, respectively. Thus, to obtain full benefit of the galactose supported xylose fermentation at acetic acid concentration at or above 12 g/L, the galactose/xylose weight ratio should be above 2.6, such as between 2.6 and 5. Thus, the co-fermentation of xylose and galactose can be greatly improved throughout the fermentation at a galactose/xylose weight ratio above 2.6, such as between 2.6 and 5, at least up to an acetic acid level of 12 g/L. The higher fermentation rate through a part of the fermentation, as shown in Fig. 8 and Fig. 9, is still obtained at higher acetic acid levels even at lower galactose/xylose ratios, but the improved rate is then only maintained in part of the fermentation as a given amount of galactose supports fermenting a lower amount of xylose at increased acetic acid levels.

Thus, the fermentation rate of xylose can be improved when simultaneously fermented with galactose, at least up to an acetic acid level of 12 g/L. provided and as long as the presence of both galactose and xylose is established and maintained in the fermentation vessel.

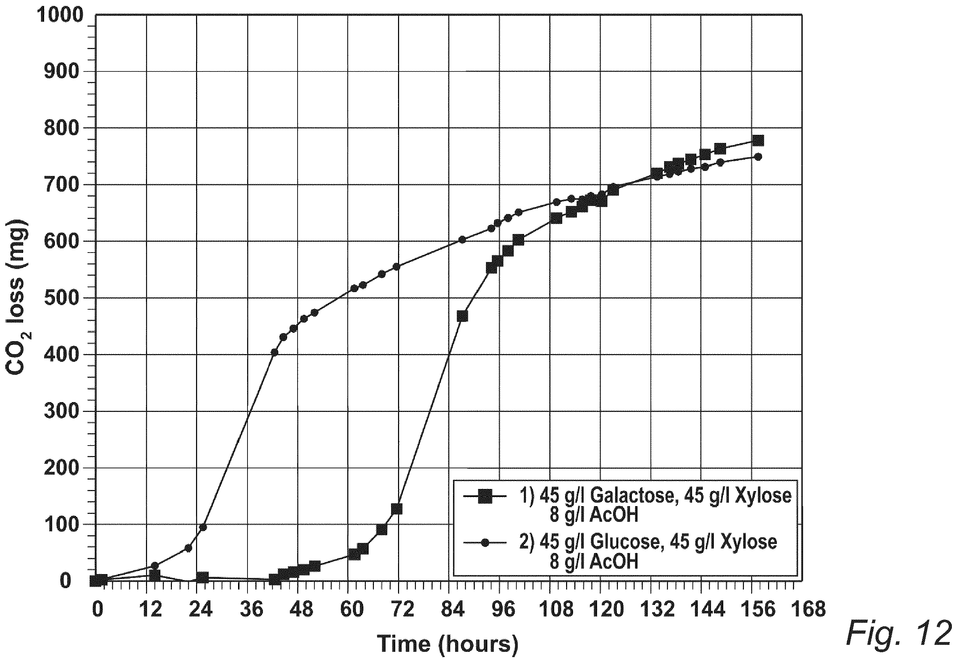

A ninth series of two measurement trials or samples is presented in the graph of Fig. 12 showing the progress of the fermentation in terms of $CO_2$ loss (in mg) over time (hours). For each of the samples, an initial yeast inoculation amount of 1 g/L was used at a moderate to relatively high amount of acetic acid at 8 g/L. The first sample in Fig. 12 is similar to the first sample of Fig. 10 (referred to as co-fermentation of xylose and galactose at a galactose/xylose weight ratio of 1), and in a second sample xylose was co-fermented with glucose at initial concentrations 45 g/L xylose and 45 g/L glucose (referred to as co-fermentation of xylose and glucose at a glucose/xylose weight ratio of 1).

It is clear from Fig. 12 that at this moderate to relatively high acetic acid concentration of 8 g/L, sample 2 with co-fermentation of xylose and glucose at a glucose/xylose weight ratio of 1 takes off much sooner (at approx. 24 hours) compared to sample 1 with co-fermentation of xylose and galactose at a galactose/xylose weight ratio of 1 (at approx. 72 hours). However, during the latter part of the fermentation process ( from 120 hours and forwards), sample 1 catches up with, and passes, sample 2. Thus, sample 2 comprising glucose has the advantage of a much shorter lag phase, caused by glucose, but when the glucose is exhausted at around 440 mg CO2 loss, there is a sharp decline in fermentation rate, which is not seen when galactose is substituted for glucose, and eventually sample 1 comprising galactose instead of glucose finishes the fermentation ahead of sample 2, because of the higher overall xylose fermentation rate. It is thus clear that the enhancement of the xylose fermentation by co-fermenting with galactose cannot be obtained by co-fermentation with glucose. The benefit obtained for xylose fermentation in co-fermentation with galactose appears unique for galactose.

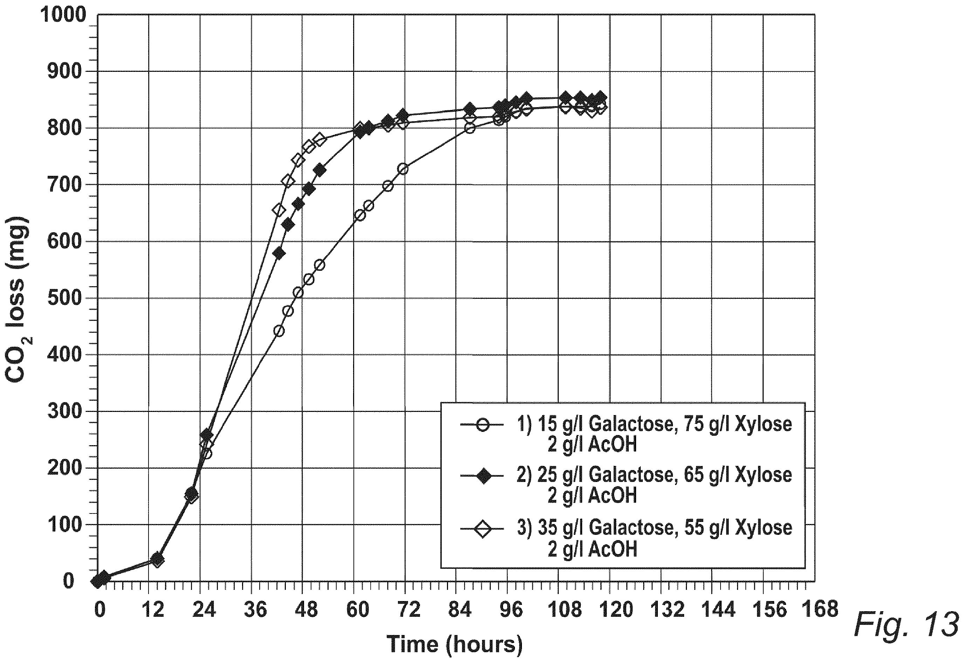

A tenth series of three measurement trials or samples is presented in the graph of Fig. 13 showing the progress of the fermentation in terms of $CO_2$ loss (in mg) over time (hours). For each of the samples, an initial yeast inoculation amount of 1 g/L was used at a relatively low amount of acetic acid at 2 g/L. In a first sample xylose was co-fermented with galactose at initial concentrations 15 g/L galactose and 75 g/L xylose (referred to as co-fermentation of galactose and xylose at a weight ratio of 0.20), in a second sample, galactose and xylose were co-fermented at initial concentrations 65 g/L xylose and 25 g/L galactose (referred to as co-fermentation of galactose and xylose at a galactose/xylose weight ratio of 0.38), and in a third sample xylose was co-fermented with galactose at initial concentrations 55 g/L xylose and 35 g/L galactose (referred to as co-fermentation of xylose and galactose at a galactose/xylose weight ratio of 0.64 ).

Fig. 13 shows that at relatively low acetic acid concentrations, to obtain maximal or near maximal effect, a relatively low galactose/xylose weight ratio is needed. Weight ratio 0.64 and weight ratio 0.38 provide for a complete fermentation of galactose and xylose at close to the same time, whereas lowering the weight ratio to 0.2 effects a proportionally larger increase of total fermentation time. We can conclude that at 2 g/l acetic acid, the maximal or near maximal effect is obtained by targeting a galactose/xylose weight ratio of approximately 0.4. It is also clear from Fig. 13, Fig. 12 and Fig. 10, that increasing the relative amount of galactose when co-fermenting galactose and xylose, improves the fermentation rate over a wide range of ratios. Thus, even though a relatively large amount of galactose may be needed to get full benefit of the positive effect of galactose supported xylose fermentation, Figs. 10, 12 and 13 clearly shows that also a small elevation of the galactose/xylose weight ratio, starting at relatively low amount of galactose (e.g. a galactose/xylose weight ratio of below 1) will have a positive effect on the xylose fermentation, as it extends the time where galactose and xylose are both present, or in other words, supports high rate xylose fermentation for a larger amount of xylose.

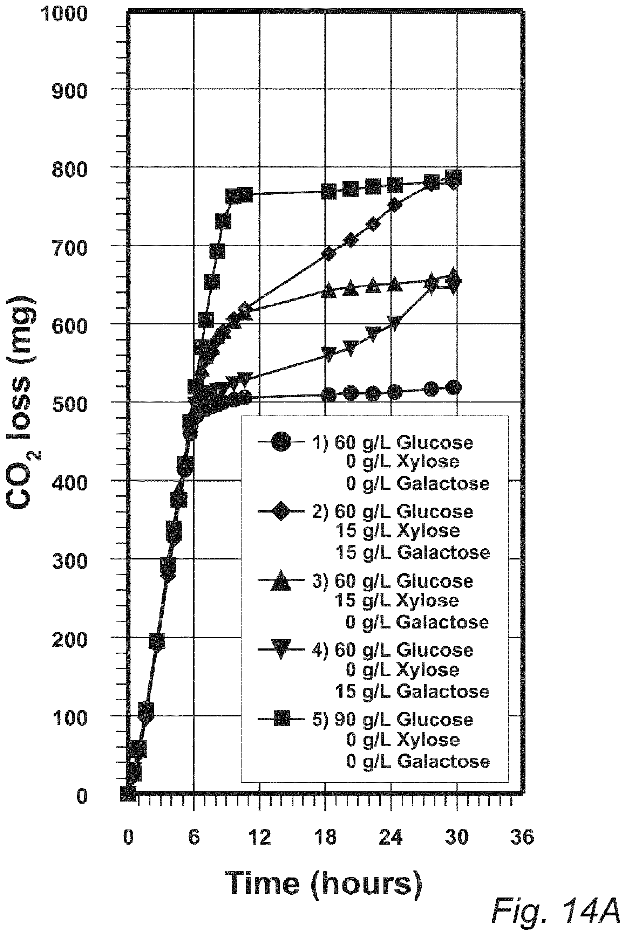

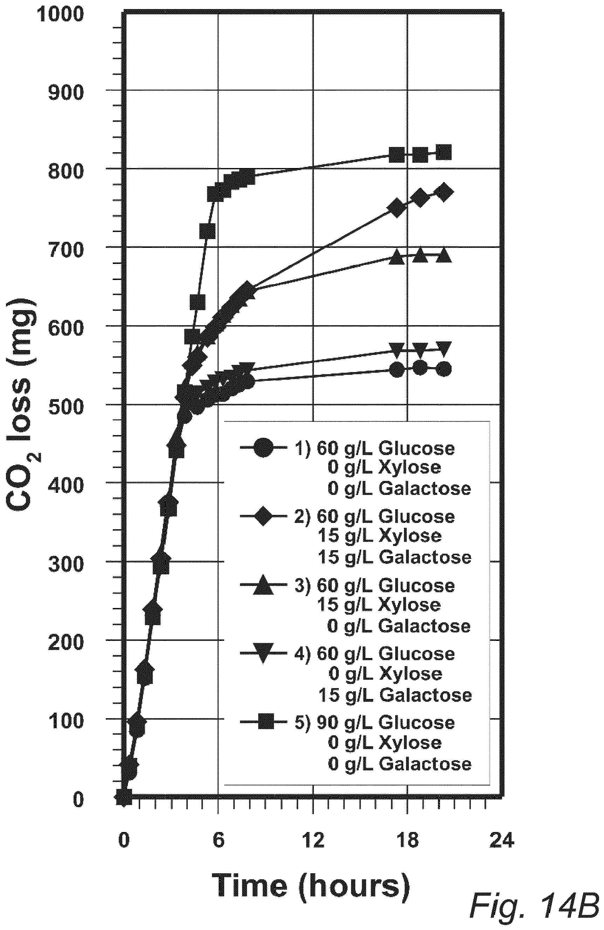

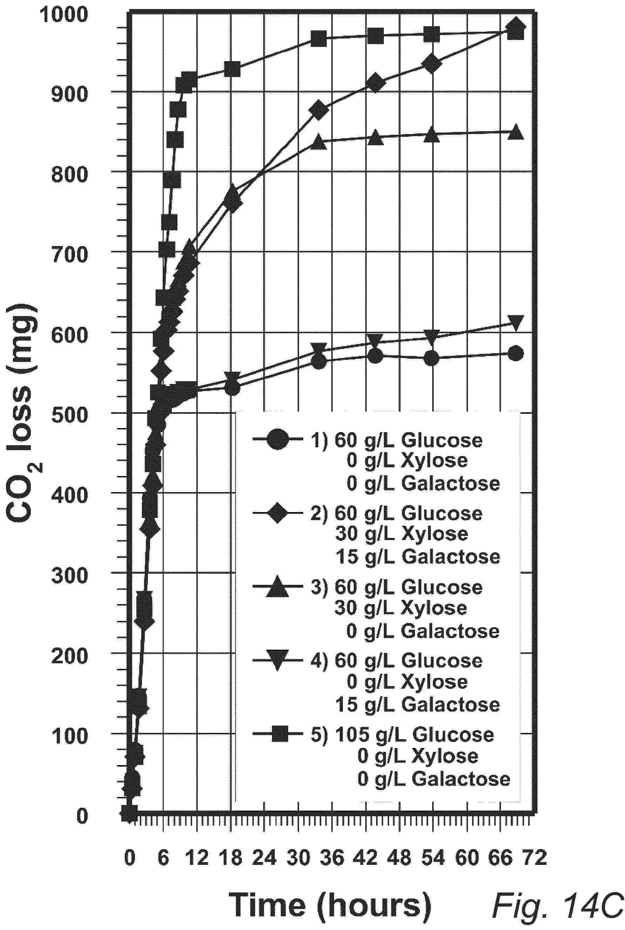

A fourteenth, fifteenth and sixteenth series, each with five measurement trials or samples, are presented in the graphs of Fig. 14A, 14B, 14C, showing the progress of the fermentation in terms of $CO_2$ loss (in mg) over time (hours). In all samples 1 only glucose was fermented at an initial concentration of 60 g/L (referred to as fermentation of glucose only 60 g/L). In all samples 2 xylose was co-fermented with galactose and glucose at initial concentrations 15 or 30 g/L xylose, 15 g/L galactose and 60 g/L glucose (referred to as co-fermentation of xylose, galactose and glucose). In all samples 3 xylose was co-fermented with glucose at initial concentrations 15 or 30 g/L xylose and 60 g/L glucose (referred to as co-fermentation of xylose and glucose). In all samples 4 galactose was co-fermented with glucose at initial concentrations 15 g/L galactose and 60 g/L glucose (referred to as co-fermentation of galactose and glucose). In all samples 5 only glucose was fermented at an initial concentration of 90 or 105 g/L (referred to as fermentation of glucose only 90 g/L or 105 g/L). Samples 1 and 5 show the end points of fermentation of 60 g/L and of 90 g/L or 105 g/L sugar respectively, samples 2-4 show the differences of supplying a glucose media with 60 g/L glucose with 15 g/L of galactose, with 15 g/L or 30 g/L of xylose, or with both galactose and xylose.

In the fourteenth series in the graph of Fig. 14A, where the yeast inoculation amount was 10 g/L yeast, it is seen that after exhaustion of 60 g/L glucose in samples 3 and 4, the yeast proceeds with fermentation of either xylose or galactose, and in sample 2 the yeast proceeds fermenting both sugars. In the fifteenth series in the graph of Fig. 14B and sixteenth series in the graph of Fig. 14C, where a higher inoculation amount of 20 g/L yeast was used, resulting in a faster glucose exhaustion, a different result is seen. In both samples 4 of the fifteenth and sixteenth series, the fermentation stops at the point where glucose is exhausted. Whereas in samples 3 of the fifteenth and sixteenth series, the yeast starts fermenting xylose right after when glucose is exhausted or about to be exhausted, and in samples 2 of the fifteenth and sixteenth series, both xylose and galactose are fermented. Or in other words, in the fifteenth and sixteenth series, galactose fermentations requires the presence of xylose, otherwise the fermentations are stuck at the glucose exhaustion point. Hence a stuck fermentation, where galactose remains unfermented, can be avoided by adding xylose or adjusting the xylose concentration in a fermentation in which an engineered yeast being able to ferment xylose is used.

Without being bound by any theory, the inventors believe that when the yeast is shifted abruptly from fermenting glucose to galactose, the fermentation may be stuck, as galactose fermentation requires prior synthesis of enzymes in a specific galactose pathway, the Leloir pathway. If the glucose is removed or exhausted before the Leloir pathway is ready, then there will not be enough energy available for synthesizing the pathway, and the yeast cannot generate such energy from galactose if that pathway is not functional. This is clear when comparing sample 3 with sample 4 for the fifteenth and sixteenth series (the fermentation of sample 3 comprising xylose exceeds the fermentation of sample 4 comprising galactose, because the fermentation stops in sample 4 without commencing with galactose consumption). The addition of xylose to the glucose-galactose mixture can alleviate such problem by bridging the gap with a sugar (xylose) that can provide energy without repressing synthesis of the galactose pathway. Hereby, full synthesis of the Leloir pathway is enabled so also the galactose will be fermented, clearly demonstrated by samples 2.

Fig. 15 shows a graph of four points determined by four of the preceding examples and figures (Fig. numbers indicated next to the points) on necessary galactose/xylose ratios to obtain full benefit at various acetic acid concentrations. The four points have been fitted to a simple exponential function of the type $y=a+b*e^{(x/c)}$, resulting in the following equation:

$$\text{Galactose(g/L)/xylose(g/L)} = 0.07 + 0.03 * e^{((\text{AcOH (g/L)})/2.35)} \quad (1)$$

The equation (1) can be generalised:

$$\text{Galactose(g/L)/xylose(g/L)} = A + B * e^{((\text{AcOH(g/L)})/C)} \quad (2)$$

The equation (1) obtained from Fig. 15 was the best fit to such an exponential function, and the resulting reference curve that describes it defines the lowest galactose/xylose weight ratio that has been shown to provide beneficial effect of galactose on xylose consumption at various acetic acid concentrations (thus, the reference curve is referred to as the minimal beneficial weight ratio curve in the figures). Stated differently, if a fermentation media, here referred to as a substrate, that has a galactose/xylose weight ratio at a certain acetic acid concentration that lies below the reference curve, and is fermented using an engineered yeast being able to ferment xylose, the xylose consumption will benefit from a raise in galactose concentration or a raise of the galactose/xylose weight ratio. But if the substrate has a galactose/xylose weight ratio at a certain acetic acid concentration that lies substantially above the reference curve, raising the galactose content will not provide any benefit to the xylose consumption.

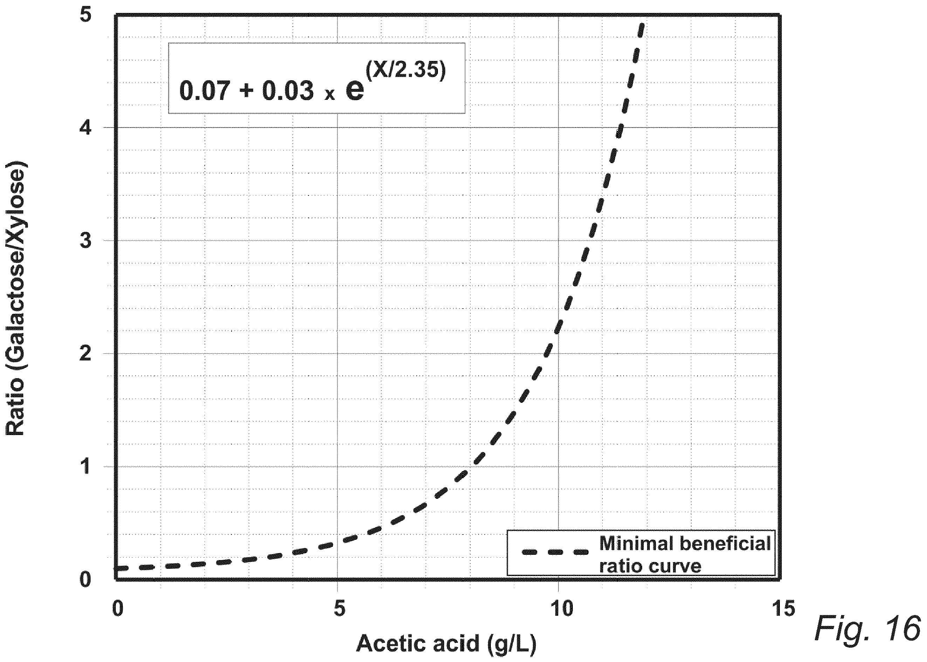

The reference curve of Fig. 15 is therefore showing the galactose/xylose weight ratio and acetic acid concentration where the galactose/xylose weight ratio is sufficient to obtain benefit of the galactose content. This reference curve, shown as a curve without the points in a coordinate system in Fig. 16, may thus be conveniently used for a determination of the possibility of optimizing the galactose/xylose weight ratio in a fermentation media having a certain acetic acid content.

A practical use of the reference curve in Fig. 15 is to evaluate whether fermentation of two different substrates will benefit from mixing the two by resulting in a benefit to the combined galactose content, instead of fermenting the substrates sequentially or in different fermentation units. When such an optimization is possible, the range of mixing ratios of the substrates providing the benefit can be determined by using the reference curve. This may for example be achieved by plotting the respective galactose/xylose weight ratio at their respective acetic acid concentration of the two substrates and in addition to that, plotting the corresponding results calculated from a row of mixtures of the two substrates.

It should be noted that undissociated acetic acid in the sample may be a factor to take into consideration, and that the dissociation degree is dependent on pH. This is well known (see for example Taherzadeh et al, 1997: Mohammad J. Taherzadeh, Claes Niklasson and Gunnar Liden,1997: "Acetic acid friend or foe in anaerobic batch conversion of glucose to ethanol by Saccharomyces cerevisiae?" Chemical Engineering Science, Vol. 52, No. 15, pp. 2653 2659). All the examples shown herein have been performed at pH 5.5, so the reference curve is valid at that pH. Change of the pH will change the dissociation proportion and thus also the concentration of the undissociated acetic acid in a calculable way according to the equation known as the Henderson-Hasselbalch equation. Thus, the equation and the reference curve shown in Fig. 15 can be adjusted to the pH accordingly (for example, the equation may be multiplied with 0.053/0.151 if the fermentation process is intended to be performed at pH 6.0 instead of pH 5.5, or with 0.360/0.151 if the fermentation process is intended to be performed at pH 5.0 instead of pH 5.5; the same calculation principle may be applied to the equation and the reference curve for any desired fermentation pH).

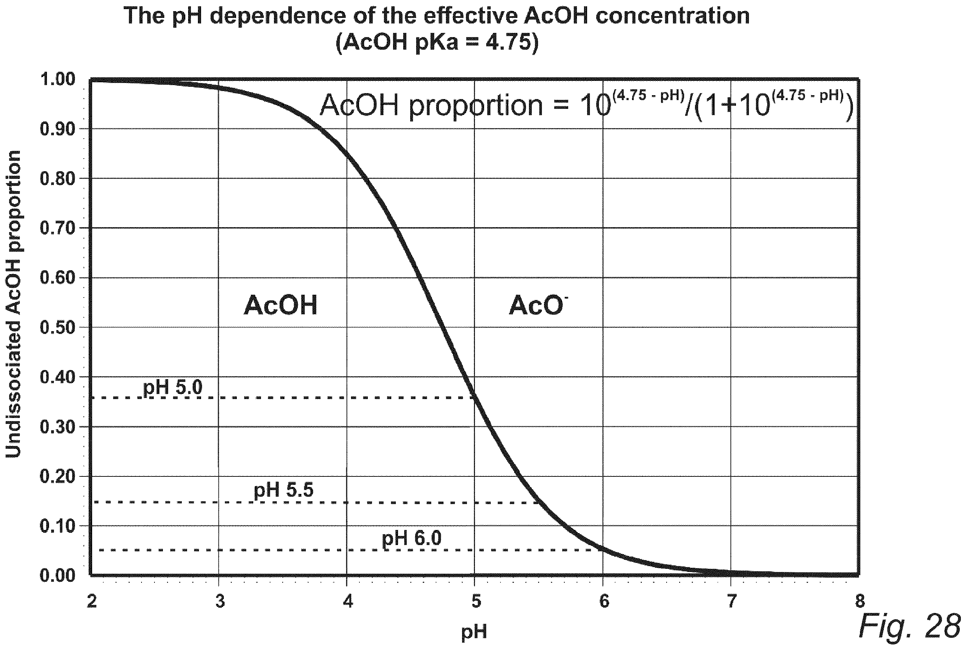

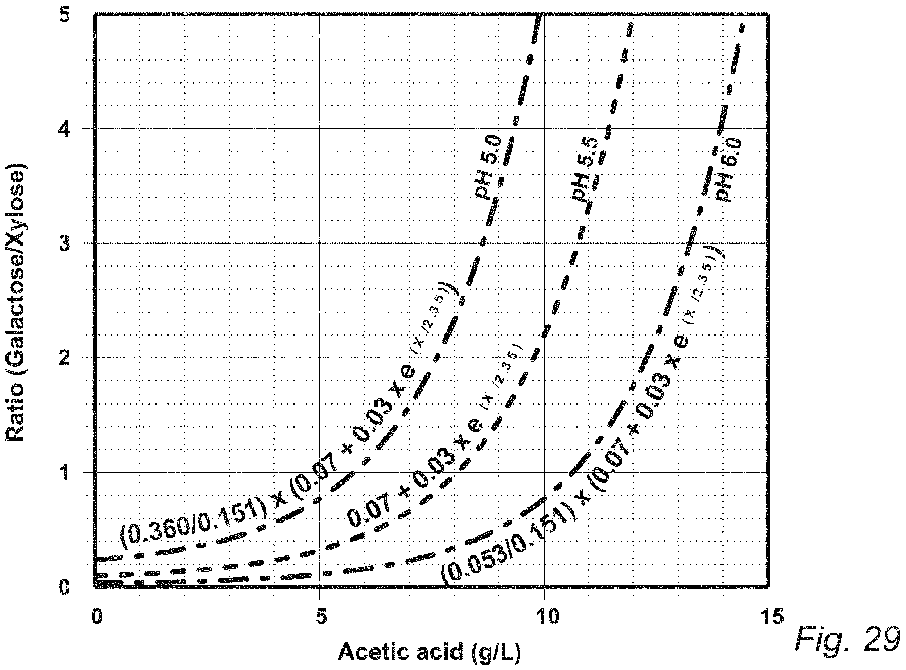

It is thus also clear that the benefit of a certain amount of galactose may be adjusted by changing the pH in the fermentation media, to the effect that increasing the pH will increase the benefit of a certain galactose amount on the xylose fermentation, and that decreasing the pH will decrease the benefit of a certain galactose amount on the xylose fermentation. The dependency of the AcOH and Ac-weight ratio on the pH of a solution is shown in Fig. 28, and the pH effect on the reference curve for fermentations at pH 5.0, pH 5.5 and pH 6.0 respectively are shown in Fig. 29.

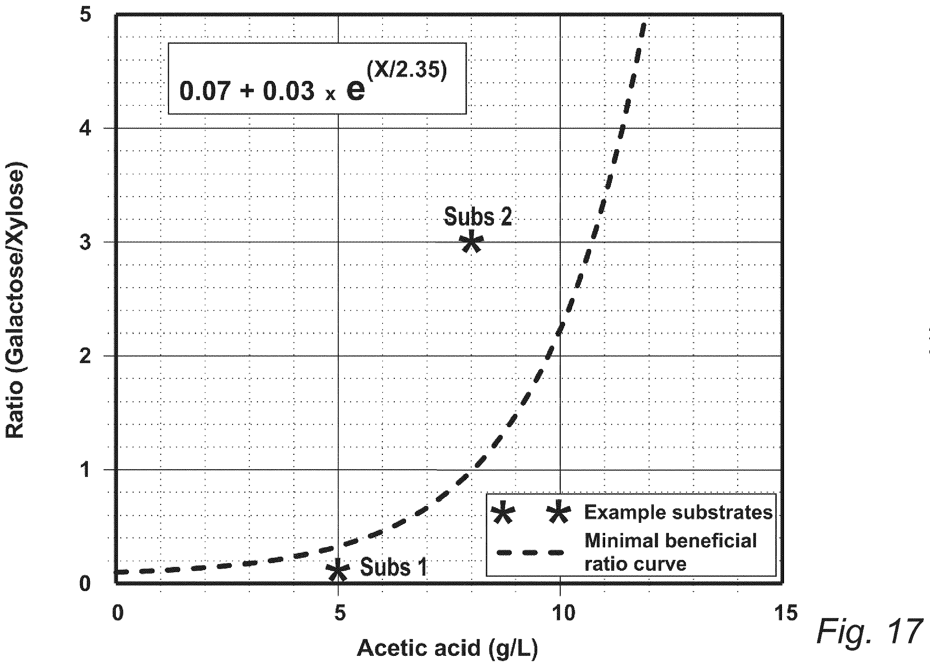

Six examples of how to use the reference curve at pH 5.5 for different substrates and various possible results are provided below in examples 1 to 6 and Figs. 17-23, using bench-mark substrates and published sugar contents in which three different sugars and the acetic acid content define the substrate. Any mixing substrates are based on weight (w/w).

In Fig. 17, two different substrates are listed in table 1 defining the substrates by three sugars, glucose, galactose and xylose, and acetic acid content, together with the respective calculated value of the galactose/xylose weight ratio.

*Table 1*

| Example 1 | Substrate 1 | Substrate 2 |
|---|---|---|
| Galactose, g/L | 5 | 30 |
| Xylose, g/L | 50 | 1C |
| Glucose, g/L | 50 | 50 |
| AcOH, g/L | 5.00 | 8.00 |
| Gal/Xyl Ratio | 0.10 | 3.00 |

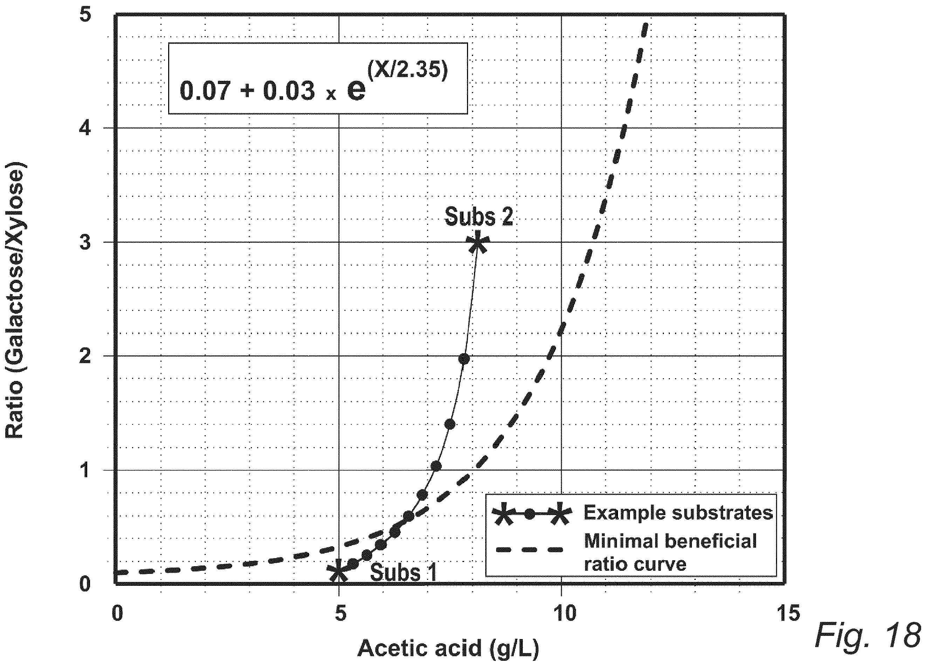

In Fig. 17, the two substrates have been plotted according to the bench-mark values of Table 1. Substrate 1 will benefit from addition of further galactose, whereas substrate 2 has a surplus of galactose. It is thus clear that by mixing substrate 1 and substrate 2, substrate 1 part will benefit from the surplus galactose in substrate 2. As the two substrates (at respective 100%) are positioned on different sides of the reference curve, i.e. substrate 1 is positioned below the reference curve and substrate 2 is positioned above the reference curve, it is also clear that at a range of mixing proportions of substrate 1 and substrate 2, the mixture ends up with a galactose/xylose weight ratio on, or above, the reference curve. Thus, there is a range of mixing proportions of substrates 1 and 2 that provides an improved use of the combined content of galactose. The beneficial proportion can be determined by calculating a set of various mixtures of substrate 1 and substrate 2 and plot the resulting galactose/xylose weight ratio as a function of the acetic acid content of those mixtures, as shown in table 2 and Fig. 18.

*Table 2*

| Example 1 | Substrate 1 | Calculated mixtures of Substrate 1 and Substrate 2 | | | | | | | | | Substrate 2 |
|---|---|---|---|---|---|---|---|---|---|---|---|
| | | 0.9 | 0.8 | 0.7 | 0.6 | 0.5 | 0.4 | 0.3 | 0.2 | 0.1 | |
| Subs 1 part | 100.000% | 90% | 80% | 70% | 60% | 50% | 40% | 30% | 20% | 10% | 0.00% |
| Subs 2 part | 0.00% | 10% | 20% | 30% | 40% | 50% | 60% | 70% | 80% | 90% | 100.00% |
| Galactose, g/L | 5 | 7.50 | 10.00 | 12.5C | 15.00 | 17.50 | 20.00 | 22.50 | 25.00 | 27.50 | 30 |
| Xylose, g/L | 50 | 46.0C | 42.00 | 38.00 | 34.00 | 30.00 | 26.00 | 22.00 | 18.00 | 14.00 | 10 |
| Glucose, g/L | 50 | 50.00 | 50.00 | 50.00 | 50.00 | 50.00 | 50.00 | 50.00 | 50.00 | 50.00 | 50 |
| AcOH, g/L | 5.00 | 5.30 | 5.60 | 5.90 | 6.20 | 6.50 | 6.80 | 7.10 | 7.40 | 7.70 | 8.00 |
| Gal/Xyl Ratio | 0.10 | 0.16 | 0.24 | 0.33 | 0.44 | 0.58 | 0.77 | 1.02 | 1.39 | 1.96 | 3.00 |

In example 1, this is shown in Fig. 18, where substrate 1, substrate 2 and the set of various mixtures are plotted in relation to the reference curve. It is clear that the function (or curve) represented by the various mixtures of substrate 1 and substrate 2 is not a straight line, and that the interception with the reference curve cannot be estimated by drawing a straight line between the point representing 100 % of substrate 1 and the point representing 100 % of substrate 2. The interception with the reference curve for example 1 is a result from a mixture of 45 % substrate 1 and 55 % substrate 2, at 6.5 g/l acetic acid (% based on weight). At such proportional mixture, a beneficial use of the combined galactose in the two substrates occur.

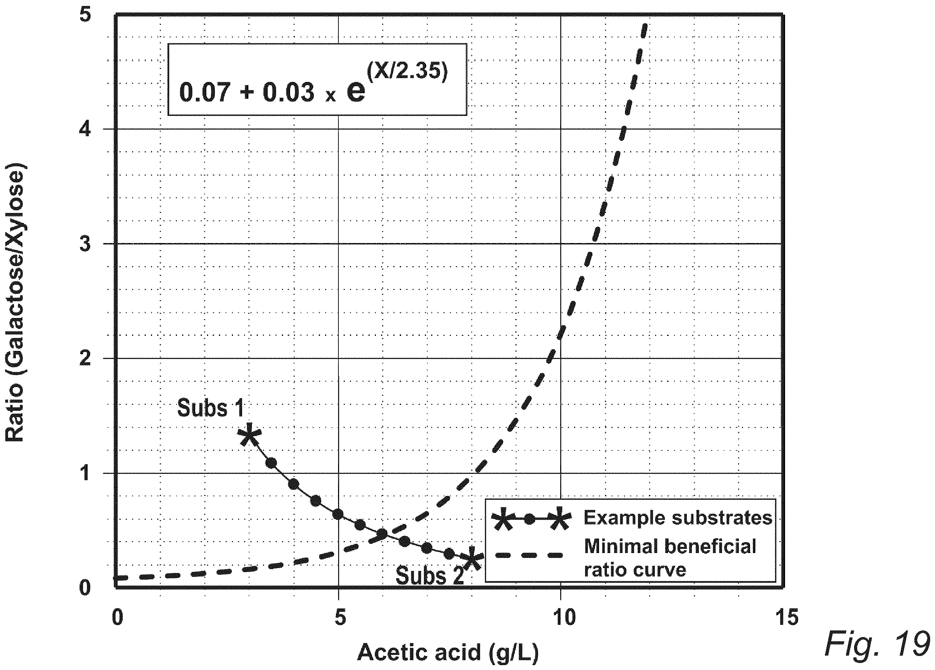

Fig. 19 shows another example of two bench-mark substrates, again referred to as substrate 1 and 2. The two different substrates of example 2, and the respective set of various calculated mixtures are listed in Table 3 and shown in Fig. 19.

*Table 3*

| **Example 2** | **Substrate 1** | Calculated mixtures of Substrate 1 and Substrate 2 | | | | | | | | | **Substrate 2** |
|---|---|---|---|---|---|---|---|---|---|---|---|
| | | 0.9 | 0.8 | 0.7 | 0.6 | 0.5 | 0.4 | 0.3 | 0.2 | 0.1 | |
| Subs 1 part | 100.00% | 90% | 80% | 70% | 60% | 50% | 40% | 30% | 20% | 10% | 0.00% |
| Subs 2 part | 0.00% | 10% | 20% | 30% | 40% | 50% | 60% | 70% | 80% | 90% | 100.00% |
| Galactose, g/L | 20 | 19.00 | 18.00 | 17.00 | 16.00 | 15.00 | 14.00 | 13.00 | 12.00 | 11.00 | 10 |
| Xylose, g/L | 15 | 17.50 | 20.00 | 22.50 | 25.00 | 27.50 | 30.00 | 32.50 | 35.00 | 37.50 | 40 |
| Glucose, g/L | 5C | 50.00 | 50.00 | 50.00 | 50.00 | 50.00 | 50.00 | 50.00 | 50.00 | 50.00 | 50 |
| AcOH, g/L | 3.00 | 3.50 | 4.0C | 4.50 | 5.00 | 5.50 | 6.00 | 6.5C | 7.00 | 7.5C | 8.00 |
| Gal/Xyl Ratio | 1.33 | 1.09 | 0.9C | 0.76 | 0.64 | 0.55 | 0.47 | 0.40 | 0.34 | 0.29 | 0.25 |

Again the substrates 1 and 2 of Table 3 are plotted in relation to the reference curve, and substrates 1 and 2 are positioned on different side of the reference curve. Thus, there is a proportional mixing of substrates 1 and 2 of example 2 that results in a galactose/xylose weight ratio as a function of the acetic acid content which ends on the reference curve. The resulting set of various mixtures of substrate 1 and 2 based on the corresponding calculations as in example 1 and Fig. 18 are shown in Fig. 19. The interception of the mixtures of the substrates in example 2 with the reference curve occur when the mixture is 65 % substrate 1 and 35 % substrate 2, at the acetic acid concentration 4.75 g/l, which mixture provides the beneficial use of the combined amount of galactose in substrate 1 and substrate 2 (% based on weight).

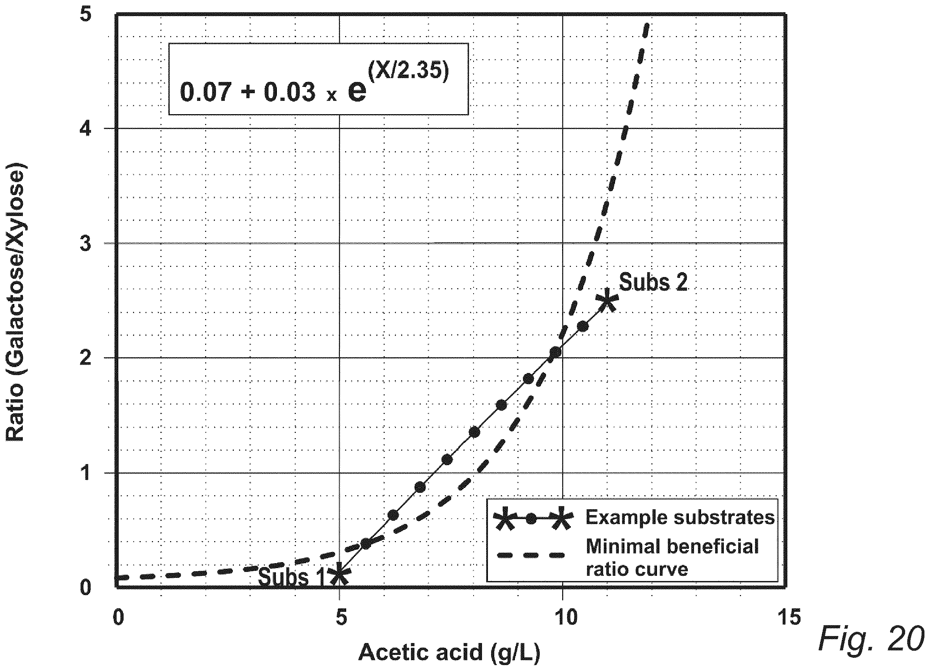

The implementation of this method described with reference to Figs. 16-19 is not restricted to a situation where the two substrates are on different sides of the reference curve. According to another example shown in Table 4 and Fig. 20, two substrates, substrate 1 and 2 of example 3, are positioned on the same side of the reference curve, here both being below the reference curve.

*Table 4*

| Example 3 | Substrate 1 | Calculated mixtures of Substrate 1 and Substrate 2 | | | | | | | | | Substrate 2 |
|---|---|---|---|---|---|---|---|---|---|---|---|
| | | 0.9 | 0.8 | 0.7 | 0.6 | 0.5 | 0.4 | 0.3 | 0.2 | 0.1 | |
| Subs 1 part | 100.00% | 90% | 80% | 70% | 60% | 50% | 40% | 30% | 20% | 10% | 0.00% |
| Subs 2 part | 0.00% | 10% | 20% | 30% | 40% | 50% | 60% | 70% | 80% | 90% | 100.00% |
| Galactose, g/L | 2 | 5.80 | 9.60 | 13.40 | 17.20 | 21.00 | 24.80 | 28.60 | 32.4C | 36.20 | 40 |
| Xylose, g/L | 15 | 15.10 | 15.20 | 15.30 | 15.40 | 15.50 | 15.60 | 15.70 | 15.80 | 15.9C | 16 |
| Glucose, g/L | 50 | 50.00 | 50.00 | 50.00 | 50.00 | 50.00 | 50.00 | 50.00 | 50.00 | 50.00 | 50 |
| AcOH, g/L | 5.00 | 5.6C | 6.20 | 6.80 | 7.40 | 8.00 | 8.60 | 9.20 | 9.80 | 10.40 | 11.00 |
| Gal/Xyl Ratio | 0.13 | 0.38 | 0.63 | 0.88 | 1.12 | 1.35 | 1.59 | 1.82 | 2.05 | 2.28 | 2.50 |

Thus, both substrates 1 and 2 would benefit from addition of galactose, as substrate 1 comprises approximately 25 % of the galactose amount that would be beneficial and the corresponding value for substrate 2 is 70 % of the beneficial galactose amount (% based on weight). Somewhat surprisingly, the corresponding set of various mixtures of substrate 1 and 2 plotted in relation to the reference curve, as listed in Table 4 and illustrated in Fig. 20, shows that by mixing two suboptimal substrates, mixtures may be achieved to obtain a better use of the galactose in both substrates when combined. The two interceptions with the reference curve are at a mixture of 75 % substrate 1 and 25 % substrate 2, as well as at a mixture of 30 % substrate 1 and 70 % substrate 2 (% based on weight). Any mixture between those two will be at or above the reference curve of the beneficial amount of galactose where xylose is fermentation is improved.

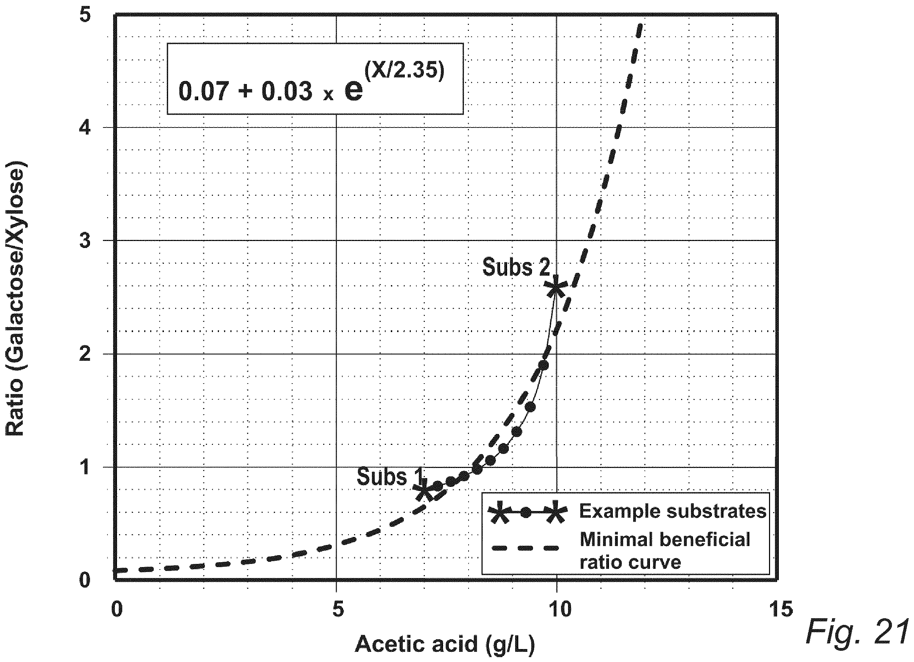

Figs. 16-20 seems to indicate that any mixture of two different substrates will be an improvement over the separate fermentation of either two substrates. This is not the case as shown in Fig. 21, where substrate 1 and substrate 2 both are plotted above the reference curve. The substrates 1 and 2 of this example (example 4) and the corresponding mixture of those two substrates is provided in Table 5 and Fig. 21.

*Table 5*

| **Example 4** | **Substrate 1** | Calculated mixtures of Substrate 1 and Substrate 2 | | | | | | | | | **Substrate 2** |
|---|---|---|---|---|---|---|---|---|---|---|---|
| | | 0.9 | 0.8 | 0.7 | 0.6 | 0.5 | 0.4 | 0.3 | 0.2 | 0.1 | |
| Subs 1 part | 100.00% | 90% | 80% | 70% | 60% | 50% | 40% | 30% | 20% | 10% | 0.00% |
| Subs 2 part | 0.00% | 10% | 20% | 30% | 40% | 50% | 60% | 70% | 80% | 90% | 100.00% |
| Galactose, g/L | 24 | 22.80 | 21.60 | 20.40 | 19.20 | 18.00 | 16.80 | 15.60 | 14.40 | 13.20 | 12 |
| Xylose, g/L | 30 | 27.46 | 24.92 | 22.38 | 19.84 | 17.30 | 14.76 | 12.22 | 9.68 | | 4.6 |
| Glucose, g/L | S | 45.46 | 40.92 | 36.38 | 31.84 | 27.30 | 22.76 | 18.22 | 13.68 | 9.14 | 4.6 |
| AcOH, g/L | 7.00 | 7.30 | 7.60 | 7.90 | 8.20 | 8.50 | 8.80 | 9.10 | 9.40 | 9.70 | 10.00 |
| Gal/Xyl Ratio | 0.80 | 0.83 | 0.87 | 0.91 | 0.97 | 1.04 | 1.14 | 1.28 | 1.49 | 1.85 | 2.61 |

The resulting function (or curve) of the substrate mixing of example 4 shows that mixing equal amounts of the two substrates that both are positioned above the reference curve may result in a mixture that ends up below the reference curve, in effect generating a fermentation media that is worse than either of the two original substrates. The method will reveal that mixing those substrates will be a disadvantage for the overall fermentation. This may be counter intuitive, but is an effect of the influence of various amounts of acetic acid on the beneficial effect of galactose

Supplementing the bench-mark examples, two examples of biomasses with published percentages of dry matter of galactose, glucose and xylose are shown here below. Acetic acid content is assumed, as this will vary according to chosen pretreatment and hydrolysis conditions. Pine and spruce species are softwood materials with relatively high galactose content and low in xylose, and wheat straw and corn stower are grassy agricultural waste materials rich in xylose and low in galactose. Table 6 shows the contents of Douglas fir and wheat straw.

*Table 6:*

| Example 5 | Douglas fir | Calculated mixtures of douglas fir and wheat straw | | | | | | | | | Wheat straw |
|---|---|---|---|---|---|---|---|---|---|---|---|
| | | 0.9 | 0.8 | 0.7 | 0.6 | 0.5 | 0.4 | | 0.3 0.2 | 0.1 | |
| Subs 1 part | 100.00% | 90% | 80% | 70% | 60% | 50% | 40% | 30% | 20% | 10% | 0.00% |
| Subs 2 part | 0.00% | 10% | 20% | 30% | 40% | 50% | 60% | 70% | 80% | 90% | 100.00% |
| Galactose, g/L | 4.7 | 4.47 | 4.24 | 4.01 | 3.78 | 3.55 | 3.32 | 3.09 | 2.86 | 2.63 | 2.4 |
| Xylose, g/L | 2.8 | 4.44 | 6.08 | 7.72 | | 11.00 | 12.64 | 14.28 | 15.92 | 17.56 | 19.2 |
| Glucose, g/L | 44 | 43.26 | 42.52 | 41.78 | 41.04 | 40.30 | 39.56 | 38.82 | 38.08 | 37.34 | 36.6 |
| AcOH, g/L | 4.00 | 4.30 | 4.60 | 4.90 | 5.20 | 5.50 | 5.80 | 6.10 | 6.40 | 6.70 | 7.00 |
| Gal/Xyl Ratio | 1.68 | 1.01 | 0.70 | 0.52 | 0.40 | 0.32 | 0.26 | 0.22 | 0.18 | 0.15 | 0.13 |

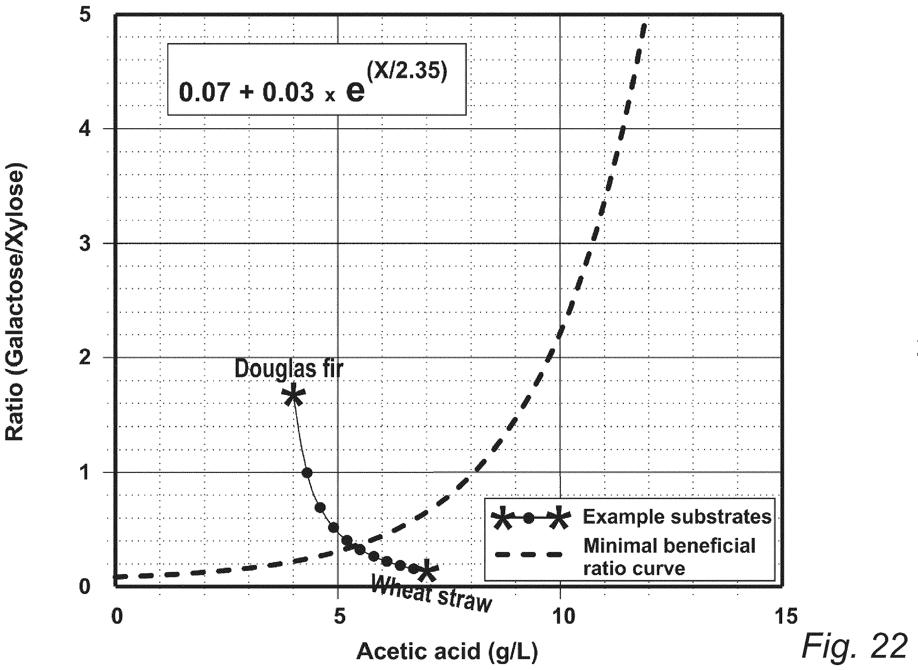

Fig. 22 shows the result of Table 6 in a graph, and the resultant function (or curve), where a 50/50 mixture of the two materials is shown to be the point where a benefit of mixing will be obtained.

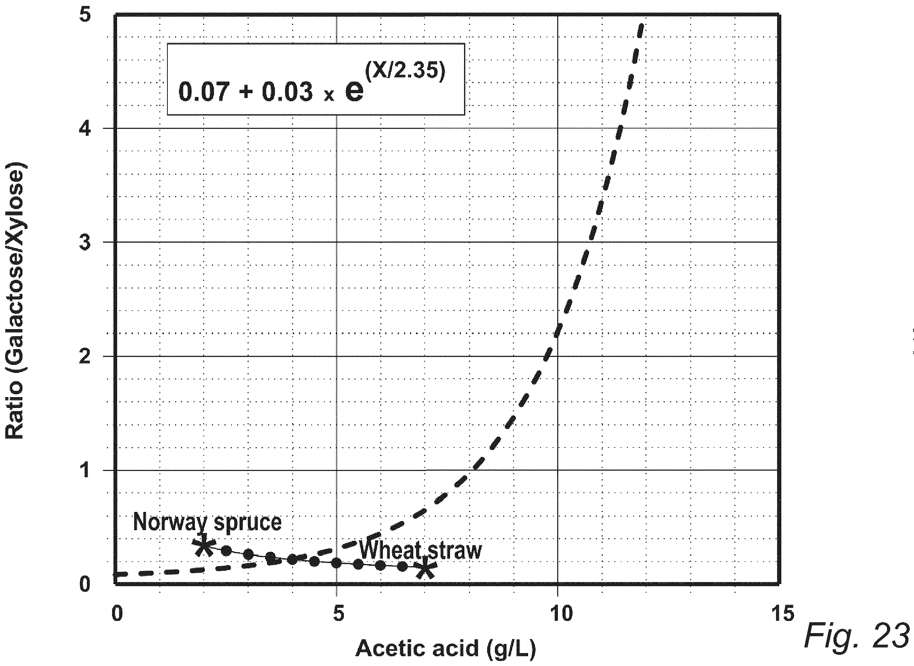

A similar example of mixing Norway spruce and wheat straw is shown in table 7 and in Fig. 23, where a similar potential mixing advantage is shown together with the table.

*Table 7*

| **Example 6** | **Norway spruce** | Calculated mixtures of Substrate 1 and Substrate 2 | | | | | | | | | **Wheat straw** |
|---|---|---|---|---|---|---|---|---|---|---|---|
| | | 0.9 | 0.8 | 0.7 | 0.6 | 0.5 | 0.4 | 0.3 | 0.2 | 0.1 | |
| Subs 1 part | 100.00% | 90% | 80% | 70% | 60% | 50% | 40% | 30% | 20% | 10% | 0.00% |
| Subs 2 part | 0.00% | 10% | 20% | 30% | 40% | 50% | 60% | 70% | 80% | 90% | 100.00% |
| Galactose, g/L | 2.3 | 2.31 | 2.32 | 2.33 | 2.34 | 2.35 | 2.36 | 2.37 | 2.38 | 2.39 | 2.4 |
| Xylose, g/L | 7.4 | 8.58 | 9.76 | 10.94 | 12.12 | 13.30 | 14.48 | 15.66 | 16.84 | 18.02 | 19.2 |
| Glucose, g/L | 4.3 | 42.36 | 41.72 | 41.08 | 40.44 | 39.80 | 39.16 | 38.52 | 37.88 | 37.24 | 36.6 |
| AcOH, g/L | 2.00 | 2.50 | 3.00 | 3.50 | 4.00 | 4.50 | 5.00 | 5.50 | 6.00 | 6.50 | 7.00 |
| Gal/Xyl Ratio | 0.31 | 0.27 | 0.24 | 0.21 | 0.19 | 0.18 | 0.16 | 0.15 | 0.14 | 0.13 | 0.13 |

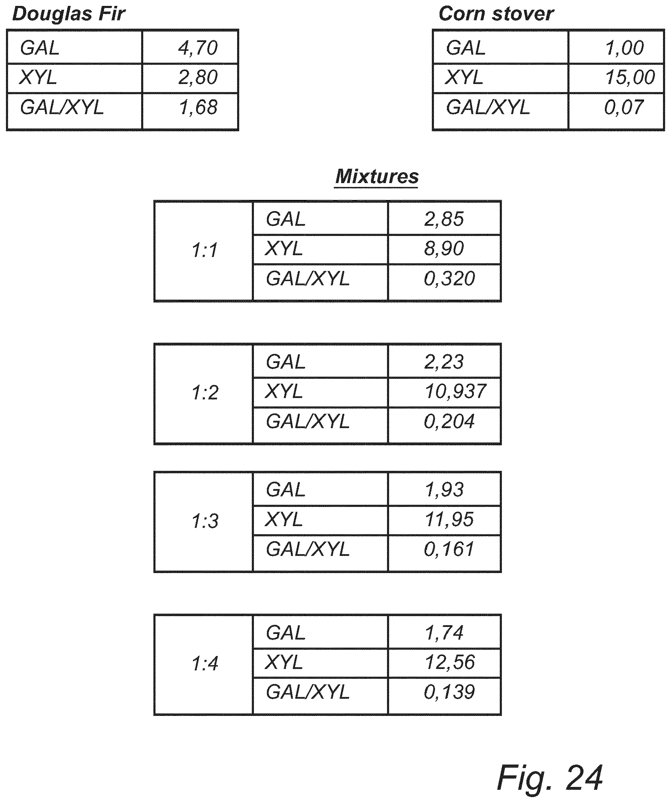

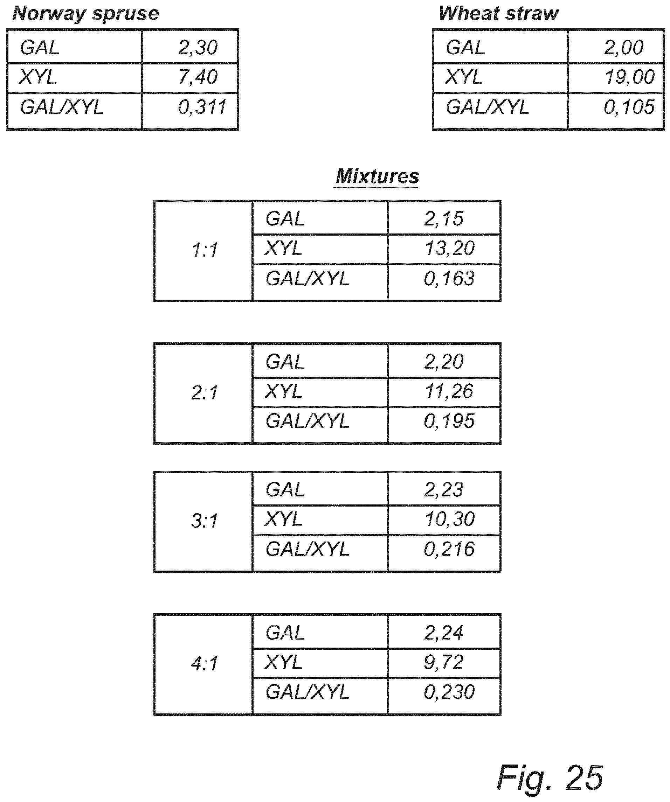

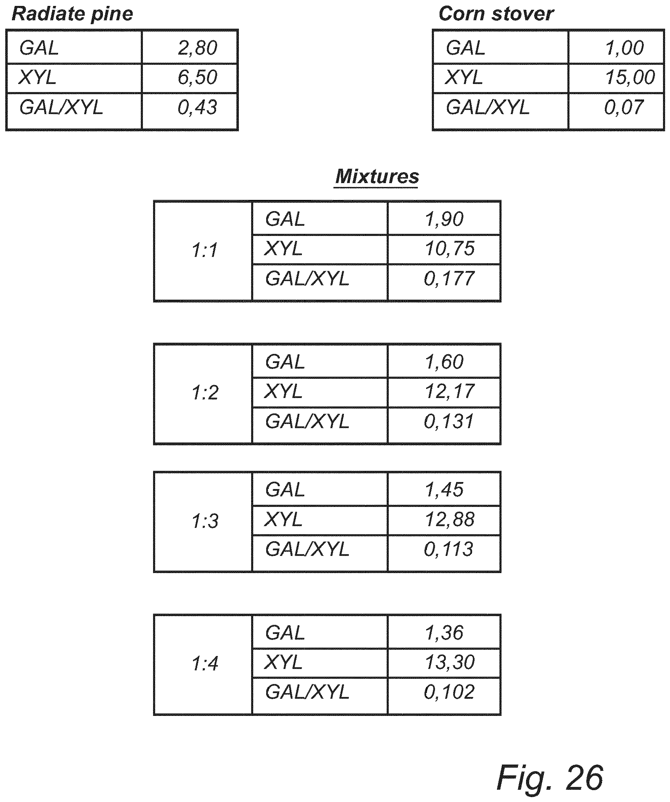

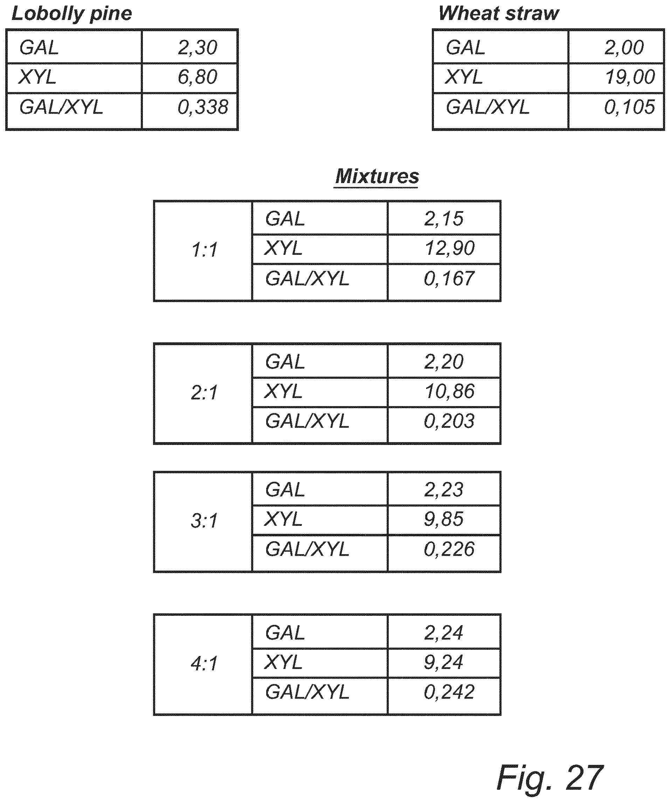

It can thus be concluded that the method of calculating the result of mixing different substrates combined with the presented knowledge of the nature and extend of the benefit provided by galactose onto the xylose fermentation may be used to foresee whether the mixing of the substrates will provide a benefit in the fermentation by resulting in an improved xylose fermentation owing to a more optimal galactose content in the mixture, or if the mixing will result in a poorer xylose fermentation performance. The method thus provides information related to foreseeing the optimal mixing proportions of the substrates, or alternatively unfavourable mixing of substrates. Figs. 24-27 show various galactose and xylose compositions for various substrates, for example making up first and second biomass-based streams. For example, the first biomass-based stream may be Douglas Fir (Fig. 24), Norway spruce (Fig. 25), Radiate pine (Fig. 26) and Lobolly pine (Fig. 27). Correspondingly, the second biomass-based stream may be Corn stover (Figs. 24 and 26) and Wheat straw (Figs. 25 and 27). For some mixtures of these first and second biomass-based streams, the weight ratio of galactose to xylose in the fermentation media will make up a beneficial combination compared to fermenting the first and second biomass-based streams separately. As disclosed earlier, any mixing resulting in a galactose to xylose weight ratio of above 0.1, such as above 0.12, or above 0.15, or above 0.166 has proven to be beneficial.

It is also clear that the method present here is not confined to the mixing of just two substrates, it may be used in a similar way to assess the effect of mixing three or more substrates as well. It should also be noted that the use of the optimized mixture of substrates may be used not only in batch fermentations, but also as the optimal mixture for feed in a fed-batch process or a repeated fed-batch process, as well as in a continuous process. The mixing of the substrates may be carried out after pretreatment and/or hydrolysis of two, or several types of biomass streams, and it may be carried out prior to pretreatment and/or hydrolysis. Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

**1.** A method for a microbial fermentation process involving fermentation of a fermentation media derived from biomass to a target fermentation product, the method comprising:

- providing a reference curve defined by a predetermined weight ratio of galactose to xylose as a function of acetic acid concentration present in the fermentation media,
- providing a first biomass-based stream and a second biomass-based stream different to the first biomass-based stream, wherein at least one of the first and second biomass-based streams has a weight ratio of galactose to xylose as a function of acetic acid concentration below the reference curve;
- mixing the first and second biomass-based streams;
- providing a fermentation media by treating the mixed first and second biomass-based streams, or treating the first and second biomass-based streams separately prior to the mixing, wherein the mixing of the first and second biomass-based streams results in a weight ratio of galactose to xylose in the fermentation media closer to the reference curve compared to at least one of the first and second biomass-based streams; and
- fermenting the fermentation media by means of fermentation microorganisms, such that the xylose and galactose are simultaneously fermented into the same target fermentation product.

**2.** The method according to claim 1, wherein the first biomass-based stream comprises galactose and xylose at a first stream galactose to xylose weight ratio, and/or wherein the second biomass-based stream comprises galactose and xylose at a second stream galactose to xylose weight ratio, and wherein the weight ratio of galactose to xylose in the fermentation media is closer to the reference curve compared to the first stream galactose to xylose weight ratio and/or the second stream galactose to xylose weight ratio.

**3.** The method according to claim 2, wherein the first stream galactose to xylose weight ratio and the second stream galactose to xylose weight ratio are arranged on opposite sides of the reference curve.

**4.** The method according to any one of claims 1-3, wherein the reference curve is defined as $y = A + B * e^{(x/C)}$ wherein y is the threshold beneficial weight ratio of galactose to xylose and x is the concentration of acetic acid (g/L) in the fermentation media, and wherein A, B and C are constants.

**5.** The method according to claim 19, wherein A is 0.07 +/- 0.06, B is 0.03 +/- 0.02 and C is 2.35 +/- 0.25.

**6.** The method according to any one of the preceding claims, further comprising:

- determining the galactose to xylose weight ratio in the fermentation media;
- in response of determining that the galactose to xylose weight ratio in the fermentation media is below the reference curve, adjusting the proportion of the first and second biomass-based streams in order to adjust the galactose to xylose weight ratio to be above the reference curve.

**7.** A method for a substantial anaerobic microbial fermentation process involving fermentation of a fermentation media derived from biomass to a target fermentation product, comprising:

- providing a fermentation media comprising at least xylose and galactose;
- fermenting the fermentation media by means of fermentation microorganisms, such that the xylose and galactose are simultaneously fermented into the same target fermentation product, wherein the fermenting is performed in the presence of acetic acid.

**8.** The method according to claim 7, further comprising:

- providing a first biomass-based stream and a second biomass-based stream different to the first biomass-based stream, the first and second biomass-based streams together comprising xylose and galactose,
- mixing the first and second biomass-based streams;
- providing a fermentation media by treating the mixed first and second biomass-based streams, or treating the first and second biomass-based streams separately prior to the mixing, wherein the mixing of the first and second biomass-based streams results in a weight ratio of galactose to xylose in the fermentation media within a target range.

**9.** The method according to any one of the preceding claims, wherein the fermenting comprises simultaneously fermenting the xylose and galactose at an improved fermentation rate compared to the average rate obtained by the corresponding individual fermentation of xylose and galactose.

**10.** The method according to any one of the preceding claims, wherein the target fermentation product is a monohydric alcohol, such as ethanol.

**11.** The method according to any one of the preceding claims, wherein the initial concentration of galactose in the fermentation media is at least 2 g/L and the initial concentration of xylose in the fermentation media is at least 2 g/L, and/or wherein the ratio of initial galactose concentration and initial xylose concentration in the fermentation media is at least 0.1, such as at least 0.12, or at least 0.15, or at least 0.166.

**12.** The method according to any one of the preceding claims, wherein the fermentation process is a continuous fermentation process, or is a fed-batch fermentation process.

**13.** The method according to any one of the preceding claims, wherein the fermentation microorganisms do not comprise an inserted galactose inducible promoter regulating expression of genes involved in pentose metabolism.

**14.** Use of xylose to improve galactose fermentation by means of fermentation microorganisms, such that the xylose and galactose are simultaneously fermented into the same target fermentation product.

**15.** Use of galactose to improve xylose fermentation by means of fermentation microorganisms, such that the xylose and galactose are simultaneously fermented into the same target fermentation product.

500
500´
501
502
503
504
505
506
507
509
511
513
515

Fig. 1

56
48
50
52
58
70
54
46
60
62
32
44
64
30
42

Fig. 2

S2
S6
S4
S7
S5
S10
S20
S30
S40

Fig. 3

Xylose, 60 g/l
Galactose, 60 g/l
Xyl & Gal, 30 g/l + 30 g/l
CO2 loss (mg)
0
100
200
300
400
500
600
Time (hours)
0
6
12
18
24
30
36
42
48


Fig. 4

CO2 loss (mg)
1000
900
800
700
600
500
400
300
200
100
0
0
6
12
18
24
30
36
42
48
54
Time (hours)
1) 10 g/l Glucose, 20 g/l Galactose
2 g/l AcOH, 60 g/L Xylose
2) 20 g/l Glucose, 10 g/l Galactose
2 g/l AcOH, 60 g/L Xylose
3) 30 g/l Glucose, 0 g/l Galactose
2 g/l AcOH, 60 g/L Xylose

*Fig. 5*

CO2 loss (mg)
1000
900
800
700
600
500
400
300
200
100
0
0
6
12
18
24
30
36
42
48
54
60
66
72
78
84
90
96
Time (hours)
1) 15 g/l Glucose, 30 g/l Galactose 6 g/l AcOH, 45 g/L Xylose
2) 30 g/l Glucose, 15 g/l Galactose 6 g/l AcOH, 45 g/L Xylose
3) 45 g/l Glucose, 0 g/l Galactose 6 g/l AcOH, 45 g/L Xylose


*Fig. 6*

1) 90 g/l xylose
2 g/l AcOH
2) 90 g/l galactose
2 g/l AcOH
3) 45 g/l xylose + 45 g/l galactose
2 g/l AcOH
$CO_2$ loss (mg)
0
100
200
300
400
500
600
700
800
900
1000
1100
Time (hours)
0
6
12
18
24
30
36
42
48
54
60
66
72


*Fig. 7*

1) 90 g/l xylose
6 g/l AcOH
2) 90 g/l galactose
6 g/l AcOH
3) 45 g/l xylose + 45 g/l galactose
6 g/l AcOH
$CO_2$ loss (mg)
0
100
200
300
400
500
600
700
800
900
1000
1100
Time (hours)
0
6
12
18
24
30
36
42
48
54
60
66
72

*Fig. 8*

1) 90 g/l xylose
8 g/l AcOH
2) 90 g/l galactose
8 g/l AcOH
3) 45 g/l xylose + 45 g/l galactose
8 g/l AcOH
$CO_2$ loss (mg)
0
100
200
300
400
500
600
700
800
900
1000
1100
Time (hours)
0
6
12
18
24
30
36
42
48
54
60
66
72


*Fig. 9*

1) 45 g/l Galactose, 45 g/l Xylose
8 g/l AcOH
2) 55 g/l Galactose, 35 g/l Xylose
8 g/l AcOH
3) 65 g/l Galactose, 25 g/l Xylose
8 g/l AcOH
4) 75 g/l Galactose, 15 g/l Xylose
8 g/l AcOH
5) 90 g/l Galactose, 0 g/l Xylose
8 g/l AcOH
CO2 loss (mg)
1000
900
800
700
600
500
400
300
200
100
0
0
12
24
36
48
60
72
84
96
108
120
132
144
156
168
Time (hours)

*Fig. 10*

1) 45 g/l Galactose, 45 g/l Xylose 12 g/l AcOH
2) 55 g/l Galactose, 35 g/l Xylose 12 g/l AcOH
3) 65 g/l Galactose, 25 g/l Xylose 12 g/l AcOH
4) 75 g/l Galactose, 15 g/l Xylose 12 g/l AcOH
5) 0 g/l Galactose, 90 g/l Xylose 12 g/l AcOH
CO2 loss (mg)
0
100
200
300
400
500
600
700
800
900
1000
Time (hours)
0
12
24
36
48
60
72
84
96
108
120
132
144
156
168


*Fig. 11*

CO2 loss (mg)
1000
900
800
700
600
500
400
300
200
100
0
0
12
24
36
48
60
72
84
96
108
120
132
144
156
168
Time (hours)
1) 45 g/l Galactose, 45 g/l Xylose 8 g/l AcOH
2) 45 g/l Glucose, 45 g/l Xylose 8 g/l AcOH


*Fig. 12*

CO2 loss (mg)
1000
900
800
700
600
500
400
300
200
100
0
0
12
24
36
48
60
72
84
96
108
120
132
144
156
168
Time (hours)
1) 15 g/l Galactose, 75 g/l Xylose 2 g/l AcOH
2) 25 g/l Galactose, 65 g/l Xylose 2 g/l AcOH
3) 35 g/l Galactose, 55 g/l Xylose 2 g/l AcOH


*Fig. 13*

1000
900
800
700
600
500
400
300
200
100
0
$CO_2$ loss (mg)
0
6
12
18
24
30
36
Time (hours)
1) 60 g/L Glucose
0 g/L Xylose
0 g/L Galactose
2) 60 g/L Glucose
15 g/L Xylose
15 g/L Galactose
3) 60 g/L Glucose
15 g/L Xylose
0 g/L Galactose
4) 60 g/L Glucose
0 g/L Xylose
15 g/L Galactose
5) 90 g/L Glucose
0 g/L Xylose
0 g/L Galactose


*Fig. 14A*

1000
900
800
700
600
500
400
300
200
100
0
$CO_2$ loss (mg)
0
6
12
18
24
Time (hours)
1) 60 g/L Glucose
0 g/L Xylose
0 g/L Galactose
2) 60 g/L Glucose
15 g/L Xylose
15 g/L Galactose
3) 60 g/L Glucose
15 g/L Xylose
0 g/L Galactose
4) 60 g/L Glucose
0 g/L Xylose
15 g/L Galactose
5) 90 g/L Glucose
0 g/L Xylose
0 g/L Galactose

*Fig. 14B*

1000
900
800
700
600
500
400
300
200
100
0
$CO_2$ loss (mg)
0 6 12 18 24 30 36 42 48 54 60 66 72
Time (hours)
1) 60 g/L Glucose
0 g/L Xylose
0 g/L Galactose
2) 60 g/L Glucose
30 g/L Xylose
15 g/L Galactose
3) 60 g/L Glucose
30 g/L Xylose
0 g/L Galactose
4) 60 g/L Glucose
0 g/L Xylose
15 g/L Galactose
5) 105 g/L Glucose
0 g/L Xylose
0 g/L Galactose

*Fig. 14C*

0.07 + 0.03 x e^(X/2.35)
5
(Fig. 12)
1.0
(Fig. 9)
0.333
(Fig. 6)
0.166
(Fig. 5)
Minimal beneficial ratio curve
Ratio (Galactose/Xylose)
Acetic acid (g/L)
0
1
2
3
4
5
0
5
10
15

*Fig. 15*

0.07 + 0.03 x e^(X/2.35)
Minimal beneficial ratio curve
Acetic acid (g/L)
Ratio (Galactose/Xylose)
0
5
10
15
1
2
3
4

*Fig. 16*

0.07 + 0.03 x e^(X/2.35)
Subs 2
Subs 1
Example substrates
Minimal beneficial ratio curve
Ratio (Galactose/Xylose)
Acetic acid (g/L)
0
1
2
3
4
5
0
5
10
15


*Fig. 17*

0.07 + 0.03 x e^(X/2.35)
Subs 2
Subs 1
Example substrates
Minimal beneficial ratio curve
Ratio (Galactose/Xylose)
Acetic acid (g/L)
0
1
2
3
4
5
0
5
10
15


*Fig. 18*

Ratio (Galactose/Xylose)
0
1
2
3
4
5
0
5
10
15
Acetic acid (g/L)
0.07 + 0.03 × e^(X/2.35)
Subs 1
Subs 2
Example substrates
Minimal beneficial ratio curve


*Fig. 19*

0.07 + 0.03 x e(X/2.35)
Subs 2
Subs 1
Example substrates
Minimal beneficial ratio curve
Ratio (Galactose/Xylose)
0
1
2
3
4
5
Acetic acid (g/L)
0
5
10
15

*Fig. 20*

Ratio (Galactose/Xylose)
0
1
2
3
4
5
Acetic acid (g/L)
0
5
10
15
0.07 + 0.03 x e^(X/2.35)
Subs 1
Subs 2
Example substrates
Minimal beneficial ratio curve


*Fig. 21*

$0.07 + 0.03 \times e^{(X/2.35)}$
Douglas fir
Wheat straw
Example substrates
Minimal beneficial ratio curve
Ratio (Galactose/Xylose)
Acetic acid (g/L)
0
1
2
3
4
5
0
5
10
15


*Fig. 22*

0.07 + 0.03 × e^(X/2.35)
Norway spruce
Wheat straw
Ratio (Galactose/Xylose)
Acetic acid (g/L)
0
1
2
3
4
5
0
5
10
15

*Fig. 23*

**Douglas Fir**

| | |
|---|---|
| GAL | 4,70 |
| XYL | 2,80 |
| GAL/XYL | 1,68 |

**Corn stover**

| | |
|---|---|
| GAL | 1,00 |
| XYL | 15,00 |
| GAL/XYL | 0,07 |

**Mixtures**

| | | |
|---|---|---|
| 1:1 | GAL | 2,85 |
| | XYL | 8,90 |
| | GAL/XYL | 0,320 |

| | | |
|---|---|---|
| 1:2 | GAL | 2,23 |
| | XYL | 10,937 |
| | GAL/XYL | 0,204 |

| | | |
|---|---|---|
| 1:3 | GAL | 1,93 |
| | XYL | 11,95 |
| | GAL/XYL | 0,161 |

| | | |
|---|---|---|
| 1:4 | GAL | 1,74 |
| | XYL | 12,56 |
| | GAL/XYL | 0,139 |

Fig. 24

**Norway spruse**

| | |
|---|---|
| GAL | 2,30 |
| XYL | 7,40 |
| GAL/XYL | 0,311 |

**Wheat straw**

| | |
|---|---|
| GAL | 2,00 |
| XYL | 19,00 |
| GAL/XYL | 0,105 |

**Mixtures**

| | | |
|---|---|---|
| 1:1 | GAL | 2,15 |
| | XYL | 13,20 |
| | GAL/XYL | 0,163 |

| | | |
|---|---|---|
| 2:1 | GAL | 2,20 |
| | XYL | 11,26 |
| | GAL/XYL | 0,195 |

| | | |
|---|---|---|
| 3:1 | GAL | 2,23 |
| | XYL | 10,30 |
| | GAL/XYL | 0,216 |

| | | |
|---|---|---|
| 4:1 | GAL | 2,24 |
| | XYL | 9,72 |
| | GAL/XYL | 0,230 |

Fig. 25

**Radiate pine**

| | |
|---|---|
| GAL | 2,80 |
| XYL | 6,50 |
| GAL/XYL | 0,43 |

**Corn stover**

| | |
|---|---|
| GAL | 1,00 |
| XYL | 15,00 |
| GAL/XYL | 0,07 |

**Mixtures**

| | | |
|---|---|---|
| 1:1 | GAL | 1,90 |
| | XYL | 10,75 |
| | GAL/XYL | 0,177 |

| | | |
|---|---|---|
| 1:2 | GAL | 1,60 |
| | XYL | 12,17 |
| | GAL/XYL | 0,131 |

| | | |
|---|---|---|
| 1:3 | GAL | 1,45 |
| | XYL | 12,88 |
| | GAL/XYL | 0,113 |

| | | |
|---|---|---|
| 1:4 | GAL | 1,36 |
| | XYL | 13,30 |
| | GAL/XYL | 0,102 |

Fig. 26

**Lobolly pine**

| | |
|---|---|
| GAL | 2,30 |
| XYL | 6,80 |
| GAL/XYL | 0,338 |

**Wheat straw**

| | |
|---|---|
| GAL | 2,00 |
| XYL | 19,00 |
| GAL/XYL | 0,105 |

**Mixtures**

| | | |
|---|---|---|
| 1:1 | GAL | 2,15 |
| | XYL | 12,90 |
| | GAL/XYL | 0,167 |

| | | |
|---|---|---|
| 2:1 | GAL | 2,20 |
| | XYL | 10,86 |
| | GAL/XYL | 0,203 |

| | | |
|---|---|---|
| 3:1 | GAL | 2,23 |
| | XYL | 9,85 |
| | GAL/XYL | 0,226 |

| | | |
|---|---|---|
| 4:1 | GAL | 2,24 |
| | XYL | 9,24 |
| | GAL/XYL | 0,242 |

Fig. 27

The pH dependence of the effective AcOH concentration
(AcOH pKa = 4.75)
AcOH proportion = 10^(4.75 - pH)/(1+10^(4.75 - pH))
AcOH
AcO-
pH 5.0
pH 5.5
pH 6.0
Undissociated AcOH proportion
1.00
0.90
0.80
0.70
0.60
0.50
0.40
0.30
0.20
0.10
0.00
2
3
4
5
6
7
8
pH

*Fig. 28*

Ratio (Galactose/Xylose)
5
4
3
2
1
0
pH 5.0
pH 5.5
pH 6.0
(0.360/0.151) x (0.07 + 0.03 x e (x /2.35))
0.07 + 0.03 x e (x /2.35)
(0.053/0.151) x (0.07 + 0.03 x e (x /2.35))
0
5
10
15
Acetic acid (g/L)


*Fig. 29*

## EUROPEAN SEARCH REPORT

Application Number
EP 21 20 7910

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X<br><br>A | US 2013/059331 A1 (KLAASSEN PAUL [NL] ET AL) 7 March 2013 (2013-03-07)<br>* abstract; figures 17-19; example 6 *<br>* paragraphs [0253] - [0256] *<br>* example 4 *<br>----- | 7,9-11, 13<br>1-6,8 | INV.<br>C12P7/10<br>C12P7/08<br>C12N1/22 |
| X<br><br>A | US 2011/027847 A1 (MATSUSHIKA AKINORI [JP] ET AL) 3 February 2011 (2011-02-03)<br>* abstract; claim 1; figure 9; example 12 *<br>----- | 7,9,10, 13<br>1-6,8 | |
| X<br><br>A | HELLE STEVE S ET AL: "Xylose fermentation by genetically modified Saccharomyces cerevisiae 259ST in spent sulfite liquor", BIORESOURCE TECHNOLOGY, ELSEVIER, AMSTERDAM, NL, vol. 92, no. 2, 27 October 2003 (2003-10-27), pages 163-171, XP085125649, ISSN: 0960-8524, DOI: 10.1016/J.BIORTECH.2003.08.011<br>* abstract; figures 5, 6 *<br>* paragraph [3.2.] *<br>* page 166 - page 167 *<br>----- | 7,9-11, 13<br><br>1-6,8 | TECHNICAL FIELDS SEARCHED (IPC)<br>C12N<br>C12P |
| X | QURESHI ET AL: "Butanol production from wheat straw by simultaneous saccharification and fermentation using Clostridium beijerinckii: Part II-Fed-batch fermentation", BIOMASS AND BIOENERGY, PERGAMON, AMSTERDAM, NL, vol. 32, no. 2, 1 February 2008 (2008-02-01), pages 176-183, XP022477164, ISSN: 0961-9534, DOI: 10.1016/J.BIOMBIOE.2007.07.005<br>* abstract; figures 2, 3 *<br>-----<br>-/-- | 7,9-13 | |

The present search report has been drawn up for all claims

| Place of search | Date of completion of the search | Examiner |
|---|---|---|
| Berlin | 25 April 2022 | Schröder, Gunnar |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

Application Number
EP 21 20 7910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/143988 A1 (MIKKELSEN MARIE JUST [DK] ET AL) 10 June 2010 (2010-06-10)<br>* paragraph [0054]; figure 6; examples 1, 4 *<br>* example 2 *<br>----- | 7,9,10, 13 | |
| X | DIETRICH KAROLIN ET AL: "Model Study To Assess Softwood Hemicellulose Hydrolysates as the Carbon Source for PHB Production in Paraburkholderia sacchari IPT 101", BIOMACROMOLECULES, vol. 19, no. 1, 8 January 2018 (2018-01-08), pages 188-200, XP055862629, US<br>ISSN: 1525-7797, DOI: 10.1021/acs.biomac.7b01446<br>Retrieved from the Internet:<br>URL:https://pubs.acs.org/doi/pdf/10.1021/acs.biomac.7b01446><br>* abstract; figure 3 *<br>* paragraph [3.3.] *<br>----- | 7,9,11, 13 | |
| | | | TECHNICAL FIELDS SEARCHED (IPC) |

The present search report has been drawn up for all claims

3

| Place of search | Date of completion of the search | Examiner |
|---|---|---|
| Berlin | 25 April 2022 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 20 7910

This annex lists the patent family members relating to the patent documents cited in the above-mentioned European search report. The members are as contained in the European Patent Office EDP file on The European Patent Office is in no way liable for these particulars which are merely given for the purpose of information.

25-04-2022

| Patent document cited in search report | Publication date | Patent family member(s) | Publication date |
|---|---|---|---|
| US 2013059331 A1 | 07-03-2013 | AR 081329 A1 | 08-08-2012 |
| | | AR 081827 A1 | 24-10-2012 |
| | | AU 2011244388 A1 | 04-10-2012 |
| | | AU 2011244395 A1 | 04-10-2012 |
| | | BR 112012027024 A2 | 06-12-2016 |
| | | CA 2794817 A1 | 27-10-2011 |
| | | CA 2800343 A1 | 27-10-2011 |
| | | CN 102869766 A | 09-01-2013 |
| | | CN 103119053 A | 22-05-2013 |
| | | DK 2561064 T3 | 15-01-2018 |
| | | EA 201201445 A1 | 30-04-2013 |
| | | EA 201201449 A1 | 29-03-2013 |
| | | EP 2560988 A1 | 27-02-2013 |
| | | EP 2561064 A1 | 27-02-2013 |
| | | EP 3241894 A1 | 08-11-2017 |
| | | ES 2651076 T3 | 24-01-2018 |
| | | JP 2013524796 A | 20-06-2013 |
| | | JP 2013524797 A | 20-06-2013 |
| | | MX 341905 B | 06-09-2016 |
| | | PL 2561064 T3 | 30-03-2018 |
| | | US 2013040297 A1 | 14-02-2013 |
| | | US 2013059331 A1 | 07-03-2013 |
| | | US 2015291983 A1 | 15-10-2015 |
| | | WO 2011131667 A1 | 27-10-2011 |
| | | WO 2011131674 A1 | 27-10-2011 |
| US 2011027847 A1 | 03-02-2011 | JP 5219074 B2 | 26-06-2013 |
| | | JP 2009195220 A | 03-09-2009 |
| | | US 2011027847 A1 | 03-02-2011 |
| | | WO 2009093630 A1 | 30-07-2009 |
| US 2010143988 A1 | 10-06-2010 | AR 061069 A1 | 30-07-2008 |
| | | AU 2007252104 A1 | 29-11-2007 |
| | | BR PI0712490 A2 | 09-10-2012 |
| | | CA 2652451 A1 | 29-11-2007 |
| | | CL 2007001469 A1 | 30-05-2008 |
| | | CN 101490242 A | 22-07-2009 |
| | | CO 6141477 A2 | 19-03-2010 |
| | | CR 10523 A | 23-03-2009 |
| | | DK 2035543 T3 | 28-10-2013 |
| | | EP 2035543 A1 | 18-03-2009 |
| | | ES 2431644 T3 | 27-11-2013 |
| | | JP 5324430 B2 | 23-10-2013 |
| | | JP 2009537156 A | 29-10-2009 |
| | | MY 149506 A | 13-09-2013 |
| | | US 2010143988 A1 | 10-06-2010 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 21 20 7910

This annex lists the patent family members relating to the patent documents cited in the above-mentioned European search report. The members are as contained in the European Patent Office EDP file on
The European Patent Office is in no way liable for these particulars which are merely given for the purpose of information.

25-04-2022

| Patent document cited in search report | Publication date | Patent family member(s) | Publication date |
|---|---|---|---|
| | | WO 2007134607 A1 | 29-11-2007 |
| | | ZA 200809878 B | 26-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **VERDUYN C ; POSTMA E ; SCHEFFERS WA ; VAN DIJKEN JP.** Effect of benzoic acid on metabolic fluxes in yeasts: a continuous-culture study on the regulation of respiration and alcoholic fermentation. *Yeast,* 1992, vol. 8 (7), 501-17
- **TAHERZADEH ; MOHAMMAD J. TAHERZADEH ; CLAES NIKLASSON ; GUNNAR LIDEN et al.** Acetic acid friend or foe in anaerobic batch conversion of glucose to ethanol by Saccharomyces cerevisiae?. *Chemical Engineering Science,* 1997, vol. 52 (15), 2653-2659